(19) Europäisches Patentamt
European Patent Office
Office européen des brevets

(11) Publication number: **0 070 376**
**A1**

(12) **EUROPEAN PATENT APPLICATION**

(21) Application number: 82104682.8

(22) Date of filing: 28.05.82

(51) Int. Cl.³: **C 07 D 231/06**, C 07 D 401/04, C 07 D 417/04, C 07 D 403/04, C 07 D 473/04, A 61 K 31/415, A 61 K 31/44, A 61 K 31/495, A 61 K 31/53 // C07D213/77, C07D241/20

(30) Priority: 13.07.81 US 282699
13.07.81 US 282698
13.07.81 US 282905
13.07.81 US 282700
13.07.81 US 282971
13.07.81 US 282972
13.07.81 US 282827
13.07.81 US 282826

(43) Date of publication of application: 26.01.83 Bulletin 83/4

(84) Designated Contracting States: AT BE CH DE FR GB IT LI NL SE

(71) Applicant: AMERICAN CYANAMID COMPANY, 1937 West Main Street P.O. Box 60, Stamford Connecticut 06904 (US)

(72) Inventor: Dusza, John Paul, 24 Convent Road, Nanuet New York 10954 (US)
Inventor: Joseph, Joseph Peter, 14 Rutherford Place, Montvale New Jersey 07645 (US)
Inventor: Bernstein, Seymour, 26 Scott Drive, New City New York 10956 (US)

(74) Representative: Wächtershäuser, Günter, Dr., Tal 29, D-8000 München 2 (DE)

(54) **Heterocyclic substituted-amino-pyrazolines.**

(57) Novel 3-amino and 3-substituted amino-1-substituted-2-pyrazolines and compositions of matter useful for meliorating the inflammation and/or the progressive joint deterioration characteristic of arthritic disease, preventing the onset of asthmatic symptoms and allergic diseases, or as analgesic, antibacterial or antifungal agents.

EP 0 070 376 A1

R. Battisti, et. al., U.S. Patent 4,149,005 (Apr, 10, 1979) discloses compounds of the formula:

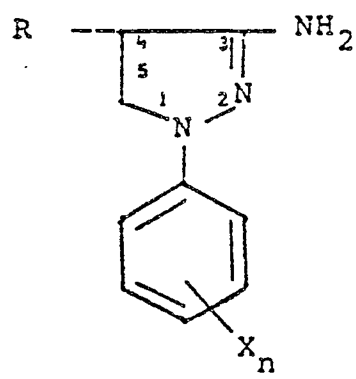

where R is H or $CH_3$, X is H, Br, Cl, alkyl, alkoxy or carboxyalkyl groups with from 1 to 4 carbon atoms or $CF_3$; and n is 1 or 2. These are disclosed as being used as intermediates in the preparation of 1-phenyl-3-aminopyrazoles as coupling components in azo dye manufacture. Related foreign patents: Ger. Offen. 2,727,706; French 2,355,834; Gr. Br. 1,515,500; Belgium 855,944; Netherland 7,706,760 and Japan 28,168.

G.A. Higgs, et. al., (Wellcome Research Laboratories); Biochemical Pharmacology, 28 1959 (1979) discloses 3-amino-1-[m-(trifluoromethyl)phenyl]-2-pyrazoline (BW 755C);

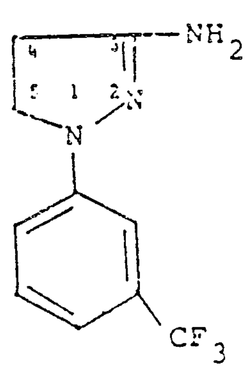

(BW 755C)

This compound is reported to have anti-inflammatory activity.

This invention relates to pyrazolines having pharmaceutical activity and represented by the following general formula:

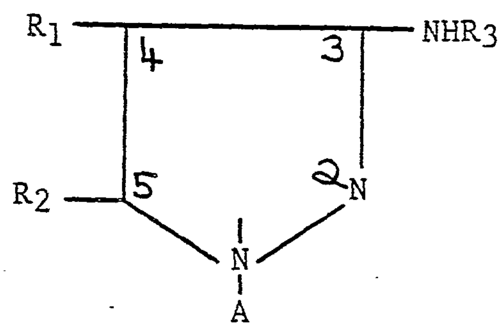

I

or a pharmacologically acceptable acid-addition salt thereof, wherein when A is

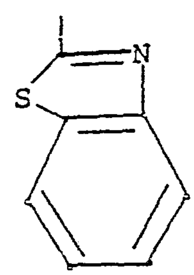

;

then $R_1$ is H; $R_2$ is phenyl or p-tolyl; and $R_3$ is $-CO-R_4$ where $R_4$ is $C_1$-$C_4$ alkyl; or when A is

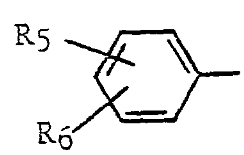

where $R_5$ and $R_6$ are the same or different and are H, Cl, F, $C_1$-$C_4$ alkyl, $-CF_3$ or $COCF_3$; then $R_1$ is H or $C_1$-$C_4$

alkyl; $R_2$ is H, $C_1$-$C_4$ alkyl, phenyl or

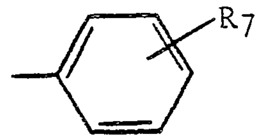

where $R_7$ is halogen; and $R_3$ is -CHO, -$COCF_3$ or -$COR_8$ where $R_8$ is $C_1$-$C_4$ alkyl; or when A is

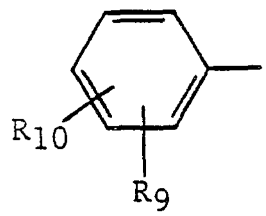

where $R_9$ and $R_{10}$ are the same or different and are H or halogen with the proviso that they cannot both be H; then $R_1$ is H or $C_1$-$C_4$ alkyl; $R_2$ is H, $C_1$-$C_4$ alkyl, phenyl or

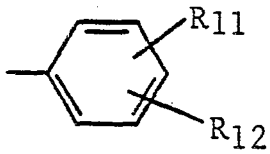

where $R_{11}$ and $R_{12}$ are the same or different and are H, halogen or $C_1$-$C_4$ alkyl with the proviso that they cannot both be H; and $R_3$ is H; or when A is

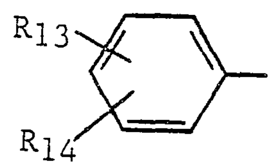

where $R_{13}$ and $R_{14}$ are H, Cl or F; then $R_1$ and $R_2$ are H or $C_1$-$C_4$ alkyl; and $R_3$ is $C_1$-$C_4$ alkyl, $CH_2CF_3$ or

$$-CH=\overset{\overset{\displaystyle COOC_2H_5}{|}}{C}-COOC_2H_5$$

; or when A is

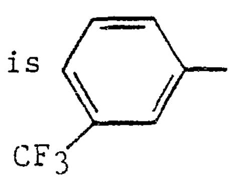

;

then $R_1$ is H or $C_1$-$C_4$ alkyl; $R_2$ is $C_1$-$C_4$ alkyl or phenyl; and $R_3$ is H; or when A is

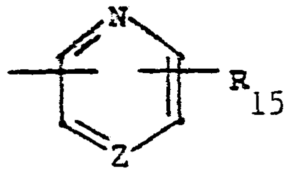 or 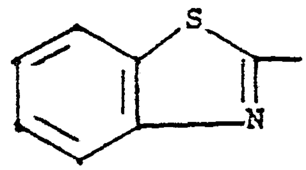

where Z is N or CH and $R_{15}$ is H, halogen or $C_1$-$C_4$ alkyl; then $R_1$ and $R_2$ are the same or different and are H, $C_1$-$C_4$ alkyl, phenyl or substituted phenyl; and $R_3$ is H with the proviso that when $R_1$ and $R_2$ are both H; then A can also be:

(1) an alkoxy-, dihalo- or nitro, halo-substituted pyridine; or

(2) a heterocycle consisting of

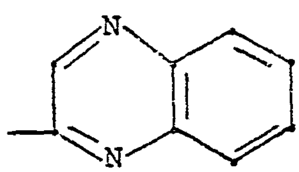 ; 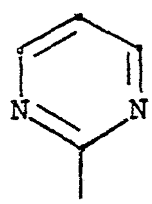 ; 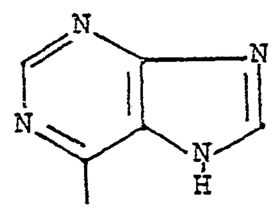 ;

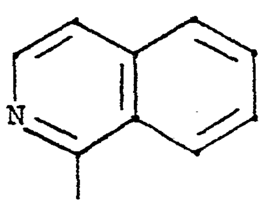 ; 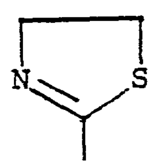 ; 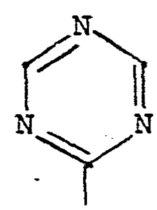 ; or

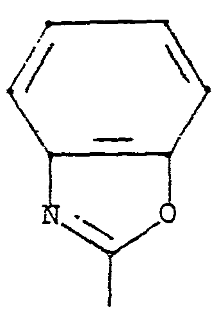

and the methyl substituted above heterocycles; or when A is

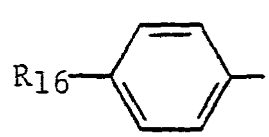

where $R_{16}$ is H, $C_1$-$C_4$ alkyl, $HO_2C$-, $CH_3O$-, $-COCF_3$ or phenyl; then $R_1$ is H or $C_1$-$C_4$ alkyl; $R_2$ is H, $C_1$-$C_4$ alkyl, phenyl or 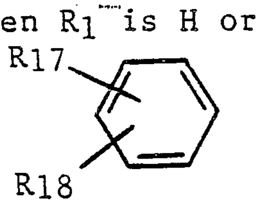

where $R_{17}$ and $R_{18}$ are the same or different and are H, halogen or $C_1$-$C_4$ alkyl with the proviso that they cannot both be H; and $R_3$ is H; or when A is 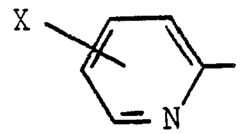

where X is halogen or $-CF_3$; then $R_1$ and $R_2$ are H or $C_1$-$C_4$ alkyl; and $R_3$ is $-COCH_3$ or -CHO.

The above compounds are all active either as anti-inflammatory agents, analgesics or as antibacterial and anti-fungal agents and in some cases are active in more than one of these areas. Some of the compounds of this invention are further useful in inhibiting the progression of arthritis such as rheumatoid arthritis and inhibiting the progression of joint deterioration or preventing the onset of asthma and other allergic diseases. They also find utility in the amelioration or prevention of pathological reactions such as osteoarthritis, gout, acute synovitis and psoriasis. The invention includes pharmaceutical compositions containing the compounds of the invention as an active ingredient.

This invention is also concerned with a composition for use in treating pain containing a quaternary salt of the formula II:

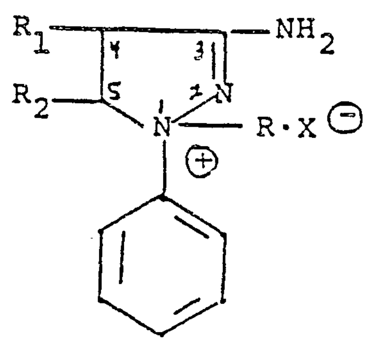

II

wherein R is $C_1$-$C_4$ alkyl; $R_1$ and $R_2$ are hydrogen or $C_1$-$C_2$ alkyl; and X is halogen.

The compounds of the present invention have been found to be highly useful for pharmaceutical therapy, when administered in amounts ranging from about 0.5 milligram to about 250 mg. per kilogram of body weight per day. A preferred dosage regimen for optimum results would be from about 5 mg. to about 100 mg. per kilogram of body weight per day, and such dosage units are employed that a total of from about 0.35 gram to about 7.0 grams of the active ingredient for a subject of about 70 kg. of body weight are administered in a 24 hour period. This dosage regimen may be adjusted to provide the optimum therapeutic response. For example, several divided doses may be administered daily or the dose may be proportionally reduced as indicated by the exigencies of the therapeutic situation. A decided practical advantage of this invention is that the active ingredient may be administered in any convenient manner such as by the oral, parenteral or topical routes. And, of course, for the control of asthma or allergic responses, the active ingredient may also be administered by inhalation.

Compositions according to the present invention have the desired clarity, stability and adaptability for parenteral use are obtained by dissolving from 0.10% to 10.0% by weight of active compound in a vehicle consisting of a polyhydric aliphatic alcohol or mixtures thereof. Especially satisfactory are glycerin, propylene glycol, and polyethylene glycols. The polyethylene glycols consist of a mixture of nonvolatile, normally liquid, polyethylene glycols which are soluble in both water and organic liquids and which have molecular weights of from about 200 to 1500. Although the amount of active compound dissolved in the above vehicle may vary from 0.10 to 10.0% by weight, it is preferred that the amount of active compound employed be from about 3.0 to about 9.0% by weight.

Although various mixtures of the aforementioned nonvolatile polyethylene glycols may be employed, it is preferred to use a mixture having an average molecular weight of from about 200 to about 400.

In addition to the active compound, the parenteral solutions may also contain various preservatives which may be used to prevent bacterial and fungal contamination. As a practical matter it is also convenient to employ antioxidants. Generally, from about 0.05 to about 0.2% concentrations of antioxidant are employed.

For intramuscular injection, the preferred concentration of active compound is 0.25 to 0.50 mg./ml. of the finished compositions. The compounds of this invention are equally adapted to intravenous administration when diluted with water or diluents employed in intravenous therapy in appropriate quantities. For intravenous use, initial concentrations down to about 0.05 to 0.25 mg./ml. of active compound are satisfactory.

The active compounds of the present invention may be orally administered, for example, with an inert diluent or with an assimilable edible carrier. They may be enclosed in hard or soft shell gelatin capsules, compressed into tablets, or incorporated directly with food. For oral therapeutic administration, the active compounds may be incorporated with excipients and used as tablets, troches, capsules, elixirs, suspensions, syrups, wafers, and the like. Such compositions and preparations should contain at least 0.1% of active compound. The percentage of the compositions and preparations may be varied and may be between about 2% to about 60% of the weight of the unit. The amount of active ingredient in such therapeutically useful compositions is such that a suitable dosage will be obtained. Preferred compositions or preparations according to the present invention are prepared so that an oral dosage unit form contains between about 50 and 250 mg. of active compound.

The tablets, troches, pills, capsules and the like may also contain the following: a binder; excipients; a disintegrating agent; a lubricant; and a sweetening or a flavoring agent. When the dosage unit form is a capsule, it may contain, in addition, a liquid carrier such as a fatty oil. Various other materials may be present as coatings or modifications of the physical form of the dosage unit. A syrup or elixir may also contain a dye. Any material used in preparing any dosage unit form should be pharmaceutically pure and substantially nontoxic in the amounts employed.

For the inhalation routes, an inhaler device may be used with the active ingredient in a suitable form such as powder or solution with appropriate pharmaceutical carriers.

Preparation of the compounds of the instant invention, which exhibit the above pharmaceutical activity, is accomplished by the adaptation of the procedure of Duffin, G. F. and Kendall, J. D., J. Chem. Soc., 1954, 408. This invention is illustrated by the following examples.

## Example 1

### 3-Amino-1-(α,α,α-trifluoro-*m*-tolyl)-2-pyrazoline

A 2.8 g. amount of sodium metal is dissolved in 125 ml. of absolute ethanol, then 21.2 g. of *m*-trifluoromethylphenylhydrazine hydrochloride is added followed by 5.5 g. of acrylonitrile. The reaction mixture is refluxed for 18 hours. The solvent is removed *in vacuo* and water is added to the residue to give a tacky solid. The solid is dissolved in dichloromethane. The solution is dried over anhydrous magensium sulfate and passed through a short column of a hydrous magnesium silicate. The effluent is collected and concentrated to separate tan crystals. This material is recrystallized from acetone-hexane to give 14.8 g. of the desired product as pale yellow needles, m.p. 104-105°C.

## Example 2

### (±)-3-Amino-4-methyl-1-(α,α,α-trifluoro-*m*-tolyl)--2-pyrazoline

A 0.7 g. amount of sodium metal is dissolved in 35.0 ml. of absolute ethanol, then 5.3 g. of *m*-trifluoromethylphenylhydrazine hydrochloride is added followed slowly by the addition of 1.7 g. of methacrylonitrile. The reaction mixture is refluxed for 18 hours, then the procedure of Example 1 is continued to give a solid. The solid is recrystallized from ether-hexane to give 3.6 g. of the desired product. A 200 mg. amount of the product is recrystallized again from ether-hexane to give 138 mg. of white crystals, m.p. 69-70°C.

Example 3

(±)-3-Amino-5-phenyl-1-(α,α,α-trifluoro-*m*-tolyl)--2-pyrazoline

The procedure of Example 2 is followed, substituting 3.2 g. of cinnamonitrile for 1.7 g. of methacrylonitrile to give a solid. The solid is recrystallized from ether-hexane to give 4.3 g. of the desired product. A 200 mg. amount of the product is again recrystallized from ether-hexane to give the product as white needles, m.p. 124-125°C.

Example 4

3-Amino-5-methyl-1-(α,α,α-trifluoro-*m*-tolyl)-

-2-pyrazoline

A 2.8 g. amount of sodium metal is dissolved in 125 ml. of absolute ethanol, then 21.2 g. of *m*-trifluoromethylphenylhydrazine hydrochloride is added followed by 6.7 g. of crotononitrile. The reaction mixture is refluxed for 6 hours. The solvent is removed *in vacuo* and water is added to separate an orange oil. The oil is dissolved in dichloromethane. The organic solution is washed with water, dried over anhyrous magnesium silicate. The effluent is evaporated to give a thin orange oil. The oil is dissolved in dichloromethane and extracted with *N* hydrochloric acid. The aqeuous acid solution is washed once with dichloromethane, then is made basic separating a light yellow oil. This oil is dissolved in dichloromethane. The organic solution is washed, dried over anhydrous magnesium sulfate, filtered and evaporated to give 8.5 g. of a pale yellow solid. The solid is recrystallized 3 times from acetone-hexane to give the product of the Example as yellow crystals, m.p. 61-63°C.

Example 5

3-Amino-5-ethyl-1-(α,α,α-trifluoro-*m*-tolyl)-
-2-pyrazoline

A 1.45 g. amount of sodium metal is dissolved in 75 ml. of absolute ethanol, then 10.5 g. of *m*-trifluoromethylphenylhydrazine hydrochloride is added followed by 4.0 g. of α-methylcrotononitrile. The reaction mixture is refluxed for 18 hours, then the procedure of Example 1 is continued to give an orange oil which is partially crystallized. This mixture is dissolved in hexane by warming. The solution is cooled and seeded. The crystals formed are collected and washed with cold hexane to give 3.4 g. of pale yellow crystals. A 300 mg. amount of the crude product is recrystallized from hexane to give 200 mg. of the desired product as white crystals, m.p. 79-80°C.

Example 6

3-Amino-1-phenyl-2-pyrazoline

A 2.0 g. amount of sodium metal is dissolved in 100 ml. of absolute ethanol, then 40.0 ml. of phenylhydrazine is added followed by 26.0 ml. of acrylonitrile. The reaction mixture is refluxed for 3 hours with exothermic crystallization of a product. The product is collected by filtration and washed with 95% ethanol. The material is recrystallized from dichloromethane-benzene to give 43.6 g. of the desired product as a solid, m.p. 168--170.5°C.

Example 7

3-Amino-1-phenyl-2-pyrazoline hydrochloride

A 2.0 g. amount of 3-amino-1-phenyl-2-pyrazoline (Example 6) is dissolved in 15 ml. of concentrated hydrochloric acid, then is poured into anhydrous ethyl ether to crystallize a product. The product is collected by filtration and recrystallized from acetone-hexane to give 1.50 g. of the desired product as a white solid, m.p. 94-96°C.

Example 8

3-Amino-5-methyl-1-phenyl-2-pyrazoline

A 2.0 g. amount of sodium metal is dissolved in 200 ml. of absolute ethanol, then 32.4 g. of phenylhydrazine in 50 ml. of ethanol is added followed in 10 minutes by 20.1 g. of crotononitrile. The reaction mixture is refluxed for 5 hours. Most of the ethanol is removed *in vacuo*, water is added and the product is collected by filtration. The solid is dissolved in dichloromethane. This solution is passed through a short column of a hydrous magnesium silicate. The column effluent is then refluxed on a steam bath with the gradual addition of hexane to crystallize a product. The product is collected and recrystallized from acetone-hexane to give 26.9 of the product of the Example as colorless prisms, m.p. 103.5-106°C.

Example 9

3-Amino-4-methyl-1-phenyl-2-pyrazoline

A 2.0 g. amount of sodium metal is dissolved in 200 ml. of absolute ethanol, then 37.4 g. of phenylhydrazine is added followed in 10 minutes by 20.1 g. of methacrylonitrile. The reaction mixture is refluxed for 4 hours then is evaporated to near dryness *in vacuo*. Water is added to the residue to separate an oil. The oil crystallizes on standing. The solid is dissolved in dichloromethane. The solution is passed through a short column of a hydrous magnesium silicate. The column effluent is evaporated to give an oil. The oil is crystallized from acetone-hexane then is recrystallized from the same solvent pair to give 20.88 g. of the desired product as colorless crystals, m.p. 83-84°C.

Example 10

3-Amino-1,4-dimethyl-1-phenyl-2-pyrazolinium iodide

A 6.0 g. amount of 3-amino-4-methyl-1-phenyl--2-pyrazoline (Example 9) and 12.0 ml. of methyl iodide is heated at reflux for 2 hours. The reaction mixture is cooled and filtered to collect 11.2 g. of crude product.

The material is dissolved in water and recrystallized from 95% ethanol to give 4.5 g. of the product of the Example as colorless needles, m.p. 168.5-170°C.

Example 11

3-Amino-1,5-diphenyl-2-pyrazoline

A 2.0 g. amount of sodium metal is dissolved in 150 ml. of absolute ethanol, then 40.0 ml. of phenylhydrazine is added followed in 5 minutes by 48.0 ml. of cinnamonitrile. The reaction mixture is refluxed for 3 hours with exothermic crystallization of a product. The product is collected by filtration and washed with water. The material is recrystallized from absolute ethanol to give 56.0 g. of the desired product as a solid, m.p. 195-197°C.

Example 12

3-Amino-1-phenyl-5-p-tolyl-2-pyrazoline

A 1.0 g. amount of sodium metal is dissolved in 100 ml. of absolute ethanol, then 20.0 ml. of phenylhydrazine is added followed in 5 minutes by 24.0 ml. of (mixed *cis* and *trans*) 4-methyl cinnamonitrile. The reaction mixture is refluxed for 4 hours then is cooled. The precipitate is collected by filtration then is recrystallized from acetone-hexane after treatment with activated charcoal to give 14.25 g. of the desired product as pale orange needles, m.p. 165-166.5°C.

Example 13

3-Amino-1-p-tolyl-2-pyrazoline

A 2.8 g. amount of sodium metal is dissolved in 150 ml. of absolute ethanol, then 15.86 g. of p-methylphenylhydrazine hydrochloride is added followed in 5 minutes by 5.5 g. of acrylonitrile. The reaction mixture is refluxed for 18 hours, then is evaporated to dryness *in vacuo*. Water is added and the separated solid is collected by filtration. The solid is dissolved in dichloromethane and passed through a short column of a hydrous magnesium silicate. The effluent is refluxed with the gradual addition of hexane to separate a crystalline product. The entire crystallization step is repeated to give

3.4 g. of the product of the Example as colorless plates, m.p. 142-143°C.

Example 14

p-(3-Amino-5-phenyl-2-pyrazolin-1-yl)benzoic acid

A 5.0 g. amount of 3-amino-1,5-diphenyl-2-pyrazoline (Example 11) and 20 ml. of trifluoroacetic anhydride is heated until solution is achieved. The reaction mixture is allowed to stand 30 minutes then the precipitate is removed by filtration. The filtrate is allowed to stand for 15 hours and the precipitate which settles out is collected and dissolved in dichloromethane. This solution is passed through a short column of a hydrous magnesium silicate. The effluent is evaporated to dryness *in vacuo*. The residue is dissolved in acetone and refluxed with the gradual addition of hexane until crystallization occurs. The mixture is cooled and filtered to give 5.3 g. of 2,2,2-trifluoro-N-[5-phenyl-1-(p-trifluoroacetylphenyl)-2-pyrazolin-3-yl]acetamide as yellow needles.

A mixture of 5.3 g. of the preceding product, 100 ml. of acetone, 100 ml. of water and 5.0 g. of pulverized potassium hydroxide is refluxed for 1.5 hours. The reaction mixture is evaporated to dryness *in vacuo*. Water is added to the residue and the mixture is extracted with dichlormethane and acidified with 5% hydrochloric acid to give a precipitate which is collected by filtration and dried. The solid is treated with ammonium hydroxide and activated charcoal and filtered. The filtrate is acidified with acetic acid to give 0.23 g. of the product of the Example as a yellow solid, m.p. 245-248°C.

Example 15

4'-(3-Amino-5-phenyl-2-pyrazolin-1-yl)-2,2,2-trifluoroacetophenone

A solution of 1.0 g. of 2,2,2-trifluoro-N-[5-phenyl-1-(p-trifluoroacetylphenyl)-2-pyrazolin-3-yl]-acetamide (prepared as described in Example 14) in 100 ml. of methanol is saturated with ammonia gas, then is stored in a refrigerator for 16 hours. The solution is evaporated to dryness in vacuo. The residue is chromatographed by preparative column chromatography using silica gel and eluting with acetone:hexane, 50:50, to collect the most polar product. The product is recrystallized from dichloromethane-hexane to yield 0.25 g. of the product of the Example as yellow needles, m.p. 214-216°C.

Example 16

3-Amino-1-(4-biphenylyl)-5-methyl-2-pyrazoline

A 5.0 g. amount of 4-amino-biphenyl is suspended in a stirred solution of 40.0 ml. of concentrated hydrochloric acid and 27.0 ml. of water maintained at 5°C. Stirring is continued and the temperature is maintained below 10°C. during the slow addition of a solution of 8.0 g. of sodium nitrite in 16.0 ml. of water. The reaction mixture is stirred for 15 minutes longer at 5°C. then is added slowly to a cooled solution (0-5°C.) of 53.0 g. of stannous chloride in 53.0 ml. of concentrated hydrochloric acid. The resulting mixture is diluted with water and treated with a 40% solution of sodium hydroxide unitl alkaline. The solid formed is collected by filtration, dissolved in dichloromethane, dried over magnesium sulfate and filtered. The filtrate is passed through a short column of a hydrous magnesium silicate. The effluent is evaporated in vacuo to give a solid. The solid is dissolved in dichloromethane. This solution is heated to boiling and hexane is added until turbidity occurs. The mixture is cooled and filtered to give 8.5 g. of 4-biphenylhydrazine as orange crystals, m.p. 135-138°C. (dec).

A 4.3 g. amount of the preceding compound is added to a solution of 0.1 g. of sodium metal dissolved in 100 ml. of absolute ethanol. Then 1.54 g. of crotononitrile is added and the mixture is refluxed for 6 hours and poured into water to separate a dark solid. The solid is collected, dissolved in dichloromethane and heated to boiling while adding hexane until turbidity results. The mixture is cooled and filtered to give 3.6 g. of the product as a solid. The recrystallization step is repeated

to give the product of the Example, m.p. 174-176°C.

Example 17

3-Amino-5-isopropyl-1-phenyl-2-pyrazoline

A mixture of 18.9 g. of isobutyraldehyde and 75.0 g. of cyanomethyltriphenylphosphorane in 500 ml. of dry benzene is refluxed with stirring under nitrogen for 6 hours. The reaction mixture solvents are removed *in vacuo* in a water bath maintained at 46-50°C. The solid is removed by filtration and washed with ether then hexane. The filtrate and washings are combined and concentrated leaving a liquid and solid. This material is distilled through a vigreaux column to give 11.03 g. of 4-methyl-2--pentenonitrile as a colorless liquid, b.p. 70-72°C/45 mm.

A 0.31 g. amount of sodium metal is dissolved in 150 ml. of absolute ethanol, then 6.3 g. of phenylhydrazine is added followed by 5.5 g. of 4-methyl-2-pentenonitrile. The reaction mixture is refluxed for 16 hours. The solvent is removed *in vacuo*, water is added to the residue and the mixture is extracted with dichloromethane. The extract is dried over anhydrous magnesium sulfate and evaporated to give a gum. The gum is chromatographed on a column containing 400 g. of a synthetic magnesium silicate absorbent. The column is eluted first with portions of 2 1/2% acetone-hexane and 5% acetone-hexane to remove exteraneous colored material, then with portions of 25% and 10% acetone-hexane to remove the product. The product cuts are evaporated to give 2.7 g. of a dark gum which solidifies on standing. This material is recrystallized twice from ether-hexane to give 1.7 g. of the product of the Example as tan crystals, m.p. 115-117°C.

Example 18

3-Amino-1-phenyl-2-pyrazoline sulfate (2:1)

A 2.0 g. amount of 3-amino-1-phenyl-2-pyrazoline (Example 6) is dissolved in 200 ml. of absolute ethanol, then 4.0 ml. of 10% w/w sulfuric acid solution is added with stirring. The reaction mixture is allowed to stand for 2 hours at room temperature then is filtered. The

precipitate is refluxed with 100 ml. of acetone, cooled and filtered to give 1.40 g. of the desired product, m.p. 181-183°C.

Example 19

3-Amino-1-phenyl-5-propyl-2-pyrazoline

A mixture of 10.8 g. of freshly distilled butyraldehyde, 30.9 g. of malonmonoamide, 25 ml. of pyridine and 5 drops of piperadine in a 100 ml. round bottom flask is heated under reflux for 24 hours in an oil bath maintained at 85-95°C. The reaction mixture is evaporated to dryness *in vacuo*. The residue is diluted with 10 ml. of water and extracted with five 50 ml. portions of ether. The extracts are combined, dried over anhydrous magnesium sulfate, filtered and evaporated to give a gummy soild. The solid is recrystallized from ether-hexane to give 4.6 g. of 2-hexenamide as white crystals, m.p. 115-117°C.

A mixture of 5.9 g. of the preceding compound (prepared as described above) and 8.7 g. of phosphorus pentoxide in a 100 ml. round bottom flask with distillation head attached is gradually heated to 200°C. in an oil bath. The mixture is heated at 200°C. for 1/2 hour, then the pressure is reduced to 50 mm. and heating is continued, gradually lowering the pressure to 15 mm. The distillate is collected in a flask cooled in dry ice to obtain 2.7 g. of 2-hexenecarbonitrile as a colorless liquid.

A 0.2 g. amount of sodium metal is dissolved in 100 ml. of absolute ethanol, then 3.1 g. of phenylhydrazine is added followed by 2.7 g. of 2-hexenecarbonitrile. The reaction mixture is refluxed for 18 hours then is evaporated to dryness *in vacuo*. The residue is dissolved in dichloromethane. The solution is washed with water, dried over anhydrous magnesium sulfate, filtered through a short column of a hydrous magnesium silicate and evaporated to dryness *in vacuo* to give a tan gum. Hexane is added and the gum solidifies. The tacky solid is collected and dried. The solid is dissolved in ether and concentrated while adding hexane to give 3.3 g. of the product of the Example

as light tan crystals, m.p. 118-120°C.

Example 20

3-Amino-1-*m*-tolyl-2-pyrazoline

A 2.8 g. amount of sodium metal is dissolved in 100 ml. of absolute ethanol, then 15.8 g. of *m*-tolylhydrazine hydrochloride is added followed in 5 minutes by 9.9 g. of β-ethoxypropionitrile. The reaction mixture is refluxed for 16 hours, then is evaporated to dryness *in vacuo*. Water is added and the precipitate is collected by filtration. The solid is dissolved in dichloromethane and is passed through a short column of a hydrous magnesium silicate. The column effluent is refluxed on a steam bath with the gradual addition of hexane to crystallize a product. The mixture is cooled and filtered to give 10.1 g. of the product of the Example as colorless crystals, m.p. 119-120°C.

Example 21

3-Amino-5-(3,4-dichlorophenyl)-1-phenyl-
-2-pyrazoline

A 0.10 g. amount of sodium metal is dissolved in 25.0 ml. of absolute ethanol, then 2.16 g. of phenylhydrazine is added, followed in 5 minutes by 3.96 g. of 3,4-dichlorocinnamonitrile. The reaction mixture is refluxed for 5 hours, then is evaporated to dryness *in vacuo*. The procedure, as described in Example 20, is continued with a second recrystallization from the same solvent pair and treatment with a hydrous magnesium silicate to give 1.35 g. of the desired product as colorless needles, m.p. 150-151.5°C.

Example 22

3-Amino-5-(*p*-chlorophenyl)-1-phenyl-
-2-pyrazoline

A mixture of 500 ml. of absolute ethanol, 5.0 ml. of 50% choline in methanol, 32.4 g. of phenylhydrazine and 49.08 g. of *p*-chlorocinnamonitrile is refluxed for 7 hours then is allowed to stand at room temperature for 16 hours, then is evaporated to dryness *in vacuo*. The procedure of Example 21 is followed to give 12.0 g. of the product of the Example as colorless needles, m.p. 183.5--185.5°C.

Example 23

3-Amino-1-(p-methoxyphenyl)-5-phenyl-2-pyrazoline

A 4.42 g. amount of sodium metal is dissolved in 300 ml. of absolute ethanol, then 27.8 g. of p-methoxyphenylhydrazine hydrochloride is added followed in 5 minutes by 20.64 g. of cinnamonitrile. The reaction mixture is refluxed for 7 hours, then is evaporated to dryness *in vacuo*. Water is added to the residue to give a sludge. The mixture is filtered and the residue is evaporated to dryness. The solid is dissolved in dichloromethane and crystallized as described in Example 21 to give 4.75 g. of the desired product as colorless needles, m.p. 163.5--166°C.

Example 24

3-Amino-5-ethyl-1-phenyl-2-pyrazoline

A 0.23 g. amount of sodium metal is dissolved in 75.0 ml. of absolute ethanol, then 10.8 g. of phenylhydrazine is added, followed in 5 minutes by 3.6 g. of α-methylcrotrononitrile. The reaction mixture is refluxed for 18 hours, then is evaporated to dryness *in vacuo*. Water is added to give a gum which solidifies on standing for 16 hours. The solid is collected and dissolved in dichloromethane. The procedure of Example 20 is continued to give 1.6 g. of the desired product as light yellow crystals, m.p. 123-125°C.

Example 25

4'-(3-Amino-4-methyl-2-pyrazolin-1-yl)--2,2,2-trifluoroacetophenone

A 5.0 g. amount of 3-amino-4-methyl-1-phenyl-2--pyrazoline (Example 9) is combined with 25 ml. of trifluoroacetic anhydride to give an exothermic reaction. The reaction mixture is allowed to stand at room temperature for 24 hours. The precipitate is collected by filtration, dissolved in dichloromethane and evaporated to dryness *in*

*vacuo*. The residue is dissolved in dichloromethane and the solution is passed through a short column of a hydrous magnesium silicate. The column effluent is refluxed on a steam bath with the gradual addition of hexane to crystallize 5.2 g. of 2,2,2-trifluoro-N-(3-methyl-1-trifluoroacetyl-2-pyrazolin-3-yl)acetamide as pale yellow needles, m.p. 202.5-204°C.

A 2.0 g. amount of the preceding compound in 150 ml. of absolute methanol is saturated at room temperature with ammonia gas, then is stored in a refrigerator for 16 hours. The reaction mixture is evaporated to dryness *in vacuo*. The residue is dissolved in dichloromethane and treated with a hydrous magnesium silicate and hexane as previously described to yield 1.6 g. of crude product. This material is chromatographed by preparative column chromatography using silica gel and eluting with acetone:hexane, 50:50, to collect the most polar product. The product is recrystallized from dichloromethane:hexane to give 0.62 g. of the product of the Example as orange needles, m.p. 159-160°C.

Example 26

3-Amino-1-(*m*-chlorophenyl)-5-phenyl-2-pyrazoline

A 0.6 g. amount of sodium metal is dissolved in 100 ml. of absolute ethanol, then 14.76 g. of *m*-chlorophenylhydrazine is added followed in 5 minutes by 12.92 g. of cinnamonitrile. The reaction mixture is refluxed for 6 hours, then cooled in an ice-bath. The precipitate formed is collected by filtration, then is dissolved in dichloromethane. This solution is passed through a short column of a hydrous magnesium silicate. The effluent is heated to reflux on a steam bath and hexane is added until a precipitate occurs. The product is collected, then is recrystallized from dichloromethane-hexane to give 7.55 g. of the product of the Example as salmon colored needles, m.p. 164-165°C.

## Example 27

### 3-Amino-1-(3,4-dichlorophenyl)-2-pyrazoline

A 2.8 g. amount of sodium metal is dissolved in 200 ml. of absolute ethanol, then 21.35 g. of 3,4-dichlorophenylhydrazine hydrochloride is added followed in 10 minutes by 5.5 g. of acrylonitrile. The reaction mixture is refluxed for 4 hours then most of the ethanol is removed *in vacuo*. Water is added to separate a solid. The solid is collected by filtration and dried. The solid is dissolved in methanol, treated with activated charcoal and filtered. The filtrate is evaporated to give a solid. The solid is recrystallized from methanol to give the desired product as tan crystals, m.p. 182-183.5°C.

## Example 28

### 3-Amino-1-(*m*-fluorophenyl)-4-methyl-2-pyrazoline

A 1.38 g. amount of sodium metal is dissolved in 150 ml. of absolute ethanol, then 8.1 g. of *m*-fluorophenylhydrazine hydrochloride is added followed by 3.9 g. of methacrylonitrile. The reaction mixture is refluxed for 5 hours, then is evaporated to dryness *in vacuo*. Water is added to give a dark semi-solid precipitate. The aqueous is decanted then more water is added and the solid is collected by filtration. The solid is dissolved in dichloromethane and the solution is passed through a short column of a hydrous magnesium silicate. The effluent is evaporated to give an oil. The oil is dissolved in hexane, treated with activated charcoal and filtered and evaporated to give a solid. The solid is recrystallized from hexane to give 0.65 g. of the desired product as light orange prisms, m.p. 78-80°C.

## Example 29

### 3-Amino-1-(*m*-fluorophenyl)-2-pyrazoline

A 1.38 g. amount of sodium metal is dissolved in 150 ml. of absolute ethanol, then 8.1 g. of *m*-fluorophenylhydrazine hydrochloride is added followed in 15 minutes by 2.75 g. of acrylonitrile. The reaction mixture is refluxed for 5 hours, then is evaporated to dryness *in vacuo*.

Water is added and the separated solid is collected by filtration. The solid is dissolved in dichloromethane and the solution is passed through a short column of a hydrous magnesium silicate. The effluent is heated to reflux on a steam bath and hexane is added to precipitate a product. The solid is collected, dissolved in acetone, treated with activated charcoal and filtered. Hexane is added to the filtrate to crystallize 2.95 g. of the product of the Example as off-white needles, m.p. 146-147°C.

Example 30

3-Amino-1-(*m*-fluorophenyl)-5-phenyl-2-pyrazoline

The procedure of Example 29 is followed substituting 6.46 g. of cinnamonitrile for 2.75 g. of acrylonitrile to give 4.75 g. of the desired product as pale yellow prisms, m.p. 165-166°C.

Example 31

3-Amino-1-(3,4-dichlorophenyl)-2-pyrazoline hydrochloride

A 1.65 g. amount of 3-amino-1-(3,4-dichlorophenyl)-2-pyrazoline (prepared as described in Example 27) is dissolved in 5.0 ml. of concentrated hydrochloric acid then methanol is added and the solvents are evaporated *in vacuo*. The residue is triturated with ether several times then acetone is added to crystallize the product. The product of the Example is collected by filtration and washed with ether to give 1.65 g. as colorless crystals, m.p. 170-175°C. (dec.).

Example 32

3-Methoxyvaleronitrile

A 248 g. amount of sodium methylate is dissolved in a solution of 828 g. of methanol diluted with 102 ml. of N,N-dimethylformamide. The above solution is added via a dropping funnel to a stirred solution of 200 g. of diethylcyanomethylphosphonate and 70.0 g. of propionaldehyde in 150 ml. of N,N-dimethylformamide, cooled in an ice-bath at 40-45°C. After the addition is complete the mixture is warmed to 50°C. and stirring is continued for one hour

without further warming or cooling.

The reaction mixture is diluted with 540 ml. of a 50:50 mixture of methanol:water, then the pH of the solution is adjusted to pH 7.0 with glacial acetic acid. The neutral solution is copiously extracted with ether and the combined ether extracts are back washed with water. The organic solution is dried over anhydrous magnesium sulfate, filtered and concentrated under reduced pressure to give an oil. The oil is distilled through a 24 inch saddle filled column, b.p. 50°C/9-10 mm. (oil bath temperature 135-145°C.), to yield a colorless liquid. The crude product is dissolved in ether and washed 3 times with water. The organic solvent is evaporated *in vacuo* to yield 26.0 of the desired product as a colorless liquid.

Example 33

2-Heptenenitrile

To 500 ml. of warm freshly distilled benzene is added 14.3 g. of valeraldehyde and 50.0 g. of (triphenylphosphoranylidene)acetonitrile. The stirred mixture is heated at reflux under nitrogen for 6 hours, then is allowed to stand at room temperature for 48 hours. The reaction solution is washed twice with 100 ml. portions of 5% sodium bisulfite and 5% sodium carbonate then is washed with water. The solution is dried over anhydrous magnesium sulfate then is evaporated to dryness leaving a solid and a thin oil. The solid is extracted several times with hexane. The combined hexane extract is distilled under vacuum (130 mm) at a temperature of 25-30°C., then the product is distilled at 70-75°C./17 mm. to collect 12.6 g. of the desired product as a colorless liquid $n_D^{22}$ 1.4382.

Example 34

2-Methylenehexanal

A mixture of 34.0 g. of dimethylamine hydrochloride, 31.8 g. of 37% formaldehyde and 35.0 g. of n--hexanal is stirred at 70°C for 24 hours. The reaction mixture is then steam distilled for 3 hours. The organic distillate is dried over anhydrous magnesium sulfate and filtered to give 31.7 g. of the desired product as a colorless liquid.

Example 35

N,O-Bis(trifluoroacetyl)hydroxylamine

To 135.2 g. of trifluoroacetic anhydride is added 14.0 g. of hydroxylamine hydrochloride. The mixture is refluxed for 2 hours then is cooled. The excess reaction solvents are removed and the residue is treated twice with hexane. The organic solvent is removed *in vacuo*. The product is recrystallized twice from dichloromethane to give 35.4 g. of the desired product as white crystals, 59-60°C. (sublim.). The product is kept dry in a vacuum desiccator.

Example 36

2-Methylenehexanenitrile

To 500 ml. of dry benzene is added 8.96 g. of 2-methylenehexanal (Example 34), 32.0 ml. of pyridine and 35.0 g. of N,O-bis(trifluoroacetyl)hydroxylamine (Example 35). The reaction mixture is refluxed for 3 hours then is cooled. The solution is washed with three 50 ml. portions of saturated sodium chloride, dried over anhydrous magnesium sulfate and filtered. The filtrate is concentrated *in vacuo* in a water bath maintained at 40-50°C. to give a yellow gum. The gum is distilled in a vigreaux column and the fraction b.p. 72-76°C./50 mm. is collected to give 3.8 g. of the desired product as a colorless liquid.

Examples 37-68

Additional 3-amino-1-halogenated phenyl-2-pyrazoline compounds listed in Table I have been prepared by following the general procedure: where 0.1 moles of sodium metal is dissolved in absolute ethanol (50--200 ml.). To this solution is added the appropriate hydrazine salt or free base (0.1 moles). The reaction mixture is refluxed (4-20 hours) after which period the solvent is removed in vacuo. The addition of water gives a filterable solid which is dissolved in dichloromethane. This solution is passed through an adsorbent column to remove impurities, then the effluent is refluxed with the gradual addition of hexane until crystallization is noted. Recrystallization from the same solvent pair (with or without another adsorbent treatment) or from acetone-hexane and/or ether-hexane provides the desired products.

## TABLE I

| Example | Compound | Hydrazine | Nitrile | M.P. °C. | Crystallization Solvent |
|---|---|---|---|---|---|
| 37 | 3-Amino-1-(3,4-dichlorophenyl)-4-methyl-2-pyrazoline | 3,4-Dichlorophenylhydrazine hydrochloride | Methacrylonitrile | 131-132.5 | Dichloromethane/Hexane |
| 38 | 3-Amino-1-(3,4-dichlorophenyl)-5-phenyl-2-pyrazoline | " | Cinnamonitrile | 166-168.5 | " |
| 39 | 3-Amino-1-(3,5-dichlorophenyl)-2-pyrazoline | 3,5-Dichlorophenylhydrazine hydrochloride | Acrylonitrile | 158-159 | Acetone/Hexane |
| 40 | 3-Amino-1,5-bis(p-chlorphenyl)-2-pyrazoline | p-Chlorophenylhydrazine hydrochloride | p-Chlorocinnamonitrile | 149-150 | " |
| 41 | 3-Amino-1-(2,5-dichlorophenyl)-2-pyrazoline | 2,5-Dichlorophenylhydrazine hydrochloride | Acrylonitrile | 136-138 | " |
| 42 | 3-Amino-1-(p-chlorophenyl)-2-pyrazoline | p-Chlorophenylhydrazine hydrochloride | " | 142.5-145 | Dichloromethane/Hexane |
| 43 | 3-Amino-1-(2,4-dichlorophenyl)-2-pyrazoline | 2,4-Dichlorophenylhydrazine hydrochloride | " | 169-172 | " |
| 44 | 3-Amino-1-(m-chlorophenyl)-2-pyrazoline | m-Chlorophenylhydrazine | " | 131-132 | Acetone/Hexane |

TABLE I (continued)

| Example | Compound | Hydrazine | Nitrile | M.P. °C. | Crystallization Solvent |
|---|---|---|---|---|---|
| 45 | 3-Amino-1-(3,4-dichlorophenyl)-5-methyl-2-pyrazoline | 3,4-Dichlorophenylhydrazine hydrochloride | Crotononitrile | 102-104 | Acetone/Hexane |
| 46 | 3-Amino-1-(p-fluorophenyl)-2-pyrazoline | p-Fluorophenylhydrazine hydrochloride | Acrylonitrile | 115-116 | Dichloromethane/-Hexane |
| 47 | 3-Amino-1-(p-fluorophenyl)-4-methyl-2-pyrazoline | " | Methacrylonitrile | 123-124 | " |
| 48 | 3-Amino-5-(p-chlorophenyl)-1-(3,4-dichlorophenyl)-2-pyrazoline | 3,4-Dichlorophenylhydrazine hydrochloride | p-Chlorocinnamonitrile | 124-126.5 | " |
| 49 | 3-Amino-5-(p-chlorophenyl)-1-(m-fluorophenyl-1-pyrazoline | m-Fluorophenylhydrazine hydrochloride | " | 135.5-136 | " |
| 50 | 3-Amino-1-(3,4-dichlorophenyl)-5-p-tolyl-2-pyrazoline | 3,4-Dichlorophenylhydrazine hydrochloride | p-Methylcinnamonitrile | 119-122 | Acetone/Hexane |
| 51 | 3-Amino-1-(p-fluorophenyl)-5-methyl-2-pyrazoline | p-Fluorophenylhydrazine hydrochloride | Crotononitrile | 133-135 | Dichloromethaen/-Hexane |

TABLE I (continued)

| Example | Compound | Hydrazine | Nitrile | M.P. °C. | Crystallization Solvent |
|---|---|---|---|---|---|
| 52 | 3-Amino-1-(m-fluorophenyl)-5-methyl-2-pyrazoline | m-Fluorophenylhydrazine hydrochloride | Crotononitrile | 65-67 | Dichloromethane/-Hexane |
| 53 | 3-Amino-1-(p-chlorophenyl)-5-methyl-2-pyrazoline | p-Chlorophenylhydrazine hydrochloride | " | 90-92 | Acetone/Hexane |
| 54 | 3-Amino-1,5-bis(3,4-dichlorophenyl)-2-pyrazoline | 3,4-Dichlorophenylhydrazine hydrochloride | 3',4'-Dichlorocinnamonitrile | 124-126 | Dichloromethane/-Hexane |
| 55 | 3-Amino-5-(p-chlorophenyl)-1-(p-fluorophenyl)-2-pyrazoline | p-Fluorophenylhydrazine hydrochloride | p-Chlorocinnamonitrile | 182-183 | Acetone/Hexane |
| 56 | 3-Amino-1-(p-chlorophenyl)-5-ethyl-2-pyrazoline | p-Chlorophenylhydrazine hydrochloride | α-Methylcrotononitrile | 117-118 | Dichloromethane/-Hexane |
| 57 | 3-Amino-1-(3,4-dichlorophenyl)-5-ethyl-2-pyrazoline | 3,4-Dichlorophenylhydrazine hydrochloride | " | 80-83 | " |
| 58 | 3-Amino-5-ethyl-1-(p-fluorophenyl)-2-pyrazoline | p-Fluorophenylhydrazine hydrochloride | " | 139-141 | " |

TABLE I (continued)

| Example | Compound | Hydrazine | Nitrile | M.P. °C. | Crystallization Solvent |
|---|---|---|---|---|---|
| 59 | 3-Amino-1-(p-chlorophenyl)-4-methyl-2-pyrazoline | p-Chlorophenylhydrazine hydrochloride | Methacrylonitrile | 107-108 | Ether/Hexane |
| 60 | 3-Amino-1-(m-chlorophenyl)-4-methyl-2-pyrazoline | m-Chlorophenylhydrazine hydrochloride | " | 85-86 | " |
| 61 | 3-Amino-5-butyl-1-(p-fluorophenyl)-2-pyrazoline | p-Fluorophenylhydrazine hydrochloride | 2-Heptenenitrile | 70-72 | Dichloromethane/-Hexane |
| 62 | 3-Amino-5-butyl-1-(p-chlorophenyl)-2-pyrazoline | p-Chlorophenylhydrazine hydrochloride | " | 94-95 | Ether/Hexane |
| 63 | 3-Amino-4-butyl-1-(p-chlorophenyl)-2-pyrazoline | " | 2-Methylenehexanenitrile | 111.5-112 | Dichloromethane/-Hexane |
| 64 | 3-Amino-1-(p-chlorophenyl)-5-propyl-2-pyrazoline | " | 2-Heptenenitrile | 90-92 | Ether/Hexane |

TABLE I (continued)

| Example | Compound | Hydrazine | Nitrile | M.P. °C. | Crystallization Solvent |
|---|---|---|---|---|---|
| 65 | 3-Amino-1-(3-chloro-p--tolyl)-5-methyl-2-pyrazoline | 3-Chloro-4-methylphenylhydrazine hydrochloride | Crotononitrile | 125-126 | Ether/Hexane |
| 66 | 3-Amino-1-(m-fluorophenyl)-5-ethyl-2-pyrazoline | m-Fluorophenylhydrazine hydrochloride | 3-Methoxyvaleronitrile | 86-88 | " |
| 67 | 3-Amino-1-(p-fluorophenyl)-5-phenyl-2-pyrazoline | p-Fluorophenylhydrazine hydrochloride | Cinnamonitrile | 160-161 | Dichloromethane/-Hexane |
| 68 | 3-Amino-1-(p-chlorophenyl)-5-phenyl-2-pyrazoline | p-Chlorophenylhydrazine hydrochloride | " | 159-161 | " |

Example 69

3-Amino-1-(p-fluorophenyl)-5-methyl-2--pyrazoline sulfate (2:1)

A mixture of 4.0 g. of 3-amino-1-(p-fluorophenyl)-5-methyl-2-pyrazoline (prepared as described in Example 51), 25.0 ml. of water and 1.0 ml. of concentrated sulfuric acid is refluxed for 6 hours. The mixture is cooled and the precipitate is collected and the solid is recrystallized first from acetone-hexane, then from acetone to give 1.0 g. of the desired product as prisms, m.p. 164.5-166.5°C.

Example 70

3-Amino-1-(3,4-dichlorophenyl)-5-methyl-2--pyrazoline sulfate (2:1)

A mixture of 1.0 g. of 3-amino-1-(3,4-dichlorophenyl)-5-methyl-2-pyrazoline (prepared as described in Example 45), 10 ml. of water and 0.5 ml. of concentrated sulfuric acid gives an immediate precipitate. The precipitate is collected and washed with dichloromethane. The material is then recrystallized from acetone-hexane to give 0.60 g. of the desired product as prisms, m.p. 160.5-163°C.

Example 71

3-Amino-1-(2,4-dichlorophenyl)-5-methyl-2--pyrazoline

A mixture of 7.8 g. of 2,4-dichlorophenylhydrazine (prepared from 2,4-dichlorohydrazine hydrochloride by treatment with 1N sodium hydroxide), 9.9 g. of crotononitrile and 3 drops of choline (45% in methanol) is heated on a steam bath for 18 hours. The solution is mixed with 200 ml. of water then 20 ml. of concentrated hydrochloric acid is added and the mixture is refluxed for 1/2 hour, cooled and made basic with 10% sodium hydroxide to give a gummy solid. The solid is placed on a chromatography column containing 300 g. of a 200 mesh synthetic magnesium silicate adsorbent. The column is eluted with 1% acetone--hexane, 5% acetone-hexane and 10% acetone-hexane to remove most of the less polar material. The elution procedure is monitored by thin layer chromoatography using the ùpper phase of a mixture of 2 parts benzene, one part acetone and 2 parts water; then the column is eluted with acetone to remove the crude product as a dark oil which partly crystallizes on standing. This material is chromatographed again using another 200 g. of the magnesium silicate adsorbent and eluting a colored impurity with 5% acetone-hexane. The column is then eluted with 50% acetone-hexane to collect a tan oil which solidifies on standing. The solid is recrystallized 3 times from ether--hexane to give 0.5 g. of the product of the Example as tan crystals, m.p. 109-111°C.

Example 72

2-(3-Amino-2-pyrazolin-1-yl)-pyridine

A 0.58 g. amount of sodium metal is dissolved in 150 ml. of absolute ethanol, then 14.0 g. of 2-hydrazinopyridine is added followed by 12.7 g. of betaethoxypropionitrile. The reaction mixture is refluxed on a steam bath for 16 hours, then the solvent is removed *in vacuo*. Water is added and the solid collected by filtration. The solid is recrystallized twice from acetone to give 5.55 g. of the product of the Example as tan colored crystals, m.p. 168.5-171°C.

Example 73

2-(3-Amino-2-pyrazolin-1-yl)-6--chloropyridine

A mixture of 100 g. of 2,6-dichloropyridine in 200 ml. of hydrazine hydrate is stirred and refluxed for 5 hours. The reaction mixture is cooled and filtered. The product is washed with water and dried to give 47.7 g. of 2-chloro-6-hydraozinopyridine, m.p. 117-119°C.

A 0.32 g. amount of sodium metal is dissolved in 100 ml. of absolute ethanol, then 10.0 g. of the product of Example 72 is added, followed by 4.0 g. of acrylonitrile. The reaction mixture is refluxed for 18 hours. The solvent is removed *in vacuo* and water is added to the

residue to separate a solid. The solid is collected by filtration, dissolved in dichloromethane, dried over magnesium sulfate, filtered through magnesium silicate and concentrated while adding hexane, to separate crystals. The mixture is cooled and filtered to give 9.3 g. of the product of the Example as a pale yellow solid, m.p. 143--145°C.

Example 74

2-Amino-1-(5-chloro-2-pyridyl)-2-pyrazoline

A mixture of 16.1 g. of 2,5-dichloropyridine in 32 ml. of hydrazine hydrate is stirred and refluxed for 15 hours. The reaction mixture is cooled and water is added. The precipitate is collected by filtration, washed with water and dried to give 12.7 g. of 5-chloro-2-hydrazinopyridine as a white solid, m.p. 127-128°C.

A 0.08 g. amount of sodium metal is dissolved in 10 ml. of absolute ethanol, then 2.5 g. of 5-chloro--2-hydrazinopyridine is added, followed by 1.70 g. of beta--ethoxypropionitrile. The procedure of Example 72 is continued to give 1.40 g. of the product of the Example as yellow crystals, m.p. 203-204.5°C.

Example 75

2-(3-Amino-4-methyl-2-pyrazolin-1-yl)--6-chloropyridine

As for Example 73 a 0.32 g. amount of sodium metal is dissolved in 100 ml. of absolute ethanol, then 10.0 g. of 2-chloro-6-hydrazinopyrdine is added, followed by 4.8 g. of methacrylonitrile in place of acrylonitrile. The procedure of Example 73 is followed to give 9.4 g. of crude product. The material is recrystallized from dichloromethane-hexane to give the product of the Example as pale yellow crystals, m.p. 175-176°C.

Example 76

2-(3-Amino-4-methyl-2-pyrazolin-1-yl)-5--chloropyridine

As for Example 75 a 0.2 g. amount of sodium metal is dissolved in 50 ml. of absolute ethanol, then 5.0 g. of 5-chloro-2-hydrazinopyridine (Example 74) is added, followed by 4.8 g. of methacrylonitrile. The reaction mixture is refluxed for 18 hours. The solvent is removed *in vacuo* and water is added to give a gum which solidifies. The solid is collected by filtration, and washed with water. The solid is dissolved in dichloromethane, dried over magnesium sulfate and filtered through anhyrous magnesium silicate. The filtrate is evaporated and the residue is triturated with hexane to give a brown solid. The solid is dissolved in dichloromethane and hexane is added while concentrating to separate a solid. The mixture is cooled and filtered to give 3.4 g. of a gray solid. The desired product is recrystallized from dichloromethane--hexane to give a white solid, m.p. 159-160°C.

Example 77

2-(3-Amino-5-methyl-2-pyrazolin-1-yl)-pyridine

A 0.3 g. amount of sodium metal is dissolved in 100 ml. of absolute ethanol, then 5.45 g. of 2-hydrazinopyridine is added, followed by 3.35 g. of crotononitrile (mixture of *cis* or *trans* isomers). The reaction mixture is refluxed for 3 hours, then the solvent is removed *in vacuo*. Water is added and the solid is collected by filtration. The solid is recrystallized from dichloromethane-hexane, then acetone-hexane to give 3.1 g. of the product of the Example as yellow crystals, m.p. 154-157°C.

Example 78

2-(3-Amino-5-methyl-2-pyrazolin-1-yl)-
-6-chloropyridine

A 0.23 g. amount of sodium metal is dissolved in 75 ml. of absolute ethanol, then 7.2 g. of 2-chloro-6-hydrazinopyridine is added followed by 3.4 g. of distilled crotononitrile. The reaction mixture is refluxed for 18 hours and the procedure of Example 76 is followed through the anhydrous magnesium silicate filtration step. The filtrate is evaporated to give a dark orange gum which crystallizes on standing. The residue is dissolved in ether, treated with activated charcoal and filtered through diatomaceous earth. The filtrate is concentrated while adding hexane at reflux to yield an oil. The oil is seeded and cooled. Ether is added and continued cooling provides light yellow crystals. The mixture is filtered to give 5.2 g. of the desired product as pale yellow crystals, m.p. 105-107°C.

Example 79

2-(3-Amino-5-methyl-2-pyrazolin-1-yl)-
-5-chloropyridine

A mixture of 0.20 g. of sodium metal dissolved in 50 ml. of absolute ethanol, 5.0 g. of 3-chloro-6-hydrazinopyridine and 4.8 g. of crotononitrile is heated at reflux for 18 hours. The procedure of Example 76 is followed through the evaporation of the magnesium silicate filtrate to give a yellow gum. Trituration with hexane gives a yellow solid. The solid is dissolved in dichloromethane. This solution is concentrated by heating on a steam bath while adding hexane, then is cooled in a refrigerator to separate 3.3 g. of the product of the Example as pale yellow crystals, m.p. 168-170°C.

Example 80

2-(3-Amino-5-methyl-2-pyrazolin-1-yl)-
-6-chloropyrazine

A 45 g. amount of 2,6-dichloropyrazine in 150 ml. of warm ethanol is slowly treated with 30 ml. of hydrazine (95+%). The mixture is refluxed for 2 hours, then evaporated *in vacuo*. The residue is shaken with 200 ml. of water and filtered. The precipitate is washed with water and air dried to give 33.0 g. of crude product. A 7.0 amount of the above product is recrystallized from 100 ml. of ethanol cooled at -10°C. The solid is collected, washed with ether and dried *in vacuo* at room temperature to give 2.8 g. of 2-chloro-6-hydrazinopyrazine, m.p. 136-137°C.

A 0.30 g. amount of sodium metal is dissolved in 100 ml. of absolute ethanol, then 6.72 g. of 2-chloro-6-hydrazinopyrazine (prepared as described above) is added and after 5 minutes, 4.6 g. of distilled crotononitrile is added. The reaction mixture is refluxed for 6 hours. The solvent is removed *in vacuo* and water is added to the residue to separate a gum. The gum is dissolved in dichloromethane, dried over magnesium sulfate and filtered. The filtrate is evaporated to give a gum. The gum is treated with benzene to separate yellow crystals. The crystals are collected, dried and recrystallized from acetone-hexane to give 1.4 g. of the desired product as yellow crystals, m.p. 147-148°C.

Example 81

2-(3-Amino-5-ethyl-2-pyrazolin-1-yl)-
-6-chloropyridine

A solution of 124 g. of sodium methylate in 414 g. of methanol, diluted with 51 ml. of N,N-dimethylformamide is added via a dropping funnel to a stirred solution of 100 g. of diethylcyanomethylphosphorate and 35 g. of freshly distilled propionaldehyde in 75 ml. of N,N-dimethylformamide maintained at 40-45°C. in an ice bath.

After the addition is complete the reaction mixture is warmed to 50°C. and stirring is continued for one hour without further external heating or cooling. The reaction mixture is diluted with 270 ml. of 50:50 methanol-water then the pH of the mixture is adjusted to a pH 7.0 with glacial acetic acid and the neutral solution is extracted thoroughly with ether. The combined ether extracts are washed with dilute acetic acid then, with water. The organic layer is dried over magnesium sulfate, filtered and concentrated in vacuo to an oil. The oil is distilled through a 2 foot saddle-filled column, giving 29.0 g. of 3-methoxyvaleronitrile, b.p. 42°C. at 3 mm., $n_D^{25°}$ 1.4190.

A 0.26 g. amount of sodium metal is dissolved in 100 ml. of absolute ethanol, then 8.1 g. of 2-chloro--6-hydrazinopyridine (Example 73) is added followed by 6.4 g. of 3-methoxyvaleronitrile. The reaction mixture is refluxed for 16 hours, then is evaporated in vacuo to give a solid. The solid is dissolved in water/dichloromethane. The organic layer is separated, dried over magnesium sulfate, filtered through anhydrous magnesium silicate and concentrated while adding hexane to yield 7.0 g. of pale yellow crystals. A 200 mg. amount of the crude product is recrystallized from dichloromethane-hexane to give 171 mg. of the product of the Example, as pale yellow crystals, m.p. 131-133°C.

Example 82

2-(3-Amino-2-pyrazolin-1-yl)benzothiazole

A mixture of 5.0 g. of β-ethoxypropionitrile, 100 ml. of absolute ethanol, 8.25 g. of 2-hydrazinobenzothazole and 2.5 g. of 50% w/v choline in methanol is refluxed on a steam bath for 16 hours, then the solvent is removed in vacuo. Water is added and the solid is collected by filtration. The solid is recrystallized twice from ethanol to give 5.75 g. of the product of the Example as grey prisms, m.p. 231.5-235°C.

Example 83

2-(3-Amino-4-methyl-2-pyrazolin-1-yl)-benzothiazole

A 0.5 g. amount of sodium metal is dissolved in 100 ml. of absolute ethanol, then 16.5 g. of 2-hydrazinobenzothiazole is added followed by 6.7 g. of methacrylonitrile. The reaction mixture is refluxed for 4 hours, then is cooled. Some of the ethanol is removed in vacuo and the precipitate is collected by filtration to give 20.6 g. of the desired product as colorless prisms, m.p. 207-209°C.

Example 84

2-(3-Amino-5-methyl-2-pyrazolin-1-yl)-benzothiazole

A mixture of 20.1 g. of crotononitrile, 80 ml. of absolute ethanol, 4.87 g. of 2-hydroazinobenzothiazole and 1.5 ml. of 50% methanolic choline is refluxed for 16 hours. The solvent is removed in vacuo and water is added. The separated solid is collected by filtration, dissolved in dichloromethane, and passed through a short column of hydrous magnesium silicate. The effluent is refluxed with the gradual addition of hexane to give a crystalline product. The crystallization step is repeated to give 0.5 g. of the product of the Example as off-white plates, m.p. 212-215°C.

Example 85

2-[3-Amino-5-(p-chlorophenyl)-2-pyrazolin-1-yl]-benzothiazole

A mixture of 3.28 g. of p-chlorocinnamonitrile, 50 ml. of absolute ethanol, 3.1 g. of 2-hydrazinobenzothiazole and 1.0 g. of 50% w/v choline in methanol is refluxed on a steam bath for 16 hours. The reaction mixture is cooled and precipitate is collected by filtration. The solid is washed with ethanol to yield 3.2 g. of crude product. This material is recrystallized from acetone to give 1.65 g. of the product of the Example as yellow crystals, m.p. 273-275°C.

Example 86

2-(3-Amino-5-p-tolyl-2-pyrazolin-1-yl)-benzothiazole

A 0.50 g. amount of sodium metal is dissolved in 250 ml. of absolute ethanol, then 8.3 g. of 2-hydrazino-benzothiazole is added followed by 7.4 g. of 4-methylcinnamonitrile. The reaction mixture is refluxed for 4 hours then is cooled. The precipitate formed is collected by filtration and wsashed with ethanol then water to give 13.7 g. of crude product. This material is recrystallized from 2-methoxyethanol, filtered and washed with hexane then to give 8.55 g. of the desired prodcut as colorless prisms, m.p. 282-285°C.

Example 87

2-(3-Amino-5-phenyl-2-pyrazolin-1-yl)-benzothiazole

A 0.46 g. amount of sodium metal is dissolved in 150 ml. of absolute ethanol, then 16.5 g. of 2-hydrazino-benzothiazole is added, followed by 12.9 g. of cinnamonitrile. The reaction mixture is refluxed for 16 hours, then is cooled. Some of the solvent is removed in vacuo and the mixture is filtered. The precipitate is washed with hexane to give 25.67 g. of crude product. A 3.0 g. amount of this material is recrystallized from acetone-hexane to give 2.35 g. of the product of the Example as buff colored prisms, m.p. 270-271.5°C.

Example 88

2-(3-Amino-5-phenyl-2-pyrazolin-1-yl)benzothiazole hydrochloride · 1/4$H_2O$

A 2.0 g. amount of 2-(3-amino-5-phenyl-2-pyrazolin-1-yl)benzothiazole (Example 77) is dissolved in 20.0 ml. of N,N-dimethylformamide. The solution is acidified with 5N ethanolic hydrochloric acid. The solvent is removed in vacuo and the residue is crystallized from ether-methanol at 5°C. to give 2.0 g. of the product of the Example as green crystals, m.p. 275-285°C.

Example 89

2-(3-Amino-5-phenyl-2-pyrazolin-1-yl)-5-chloropyridine

A 0.5 g. amount of sodium metal is dissolved in 150 ml. of absolute ethanol, then 14.36 g. of 5-chloro--2-hydrazinopyridine (prepared as described in Example 74) is added followed by 12.92 g. of cinnamonitrile. The reaction mixture is refluxed for 16 hours. The solvent is removed *in vacuo* and water is added to the residue to separate a solid. The solid is collected by filtration and dissolved in dichloromethane. The solution is dried over anhydrous sodium sulfate and filtered through a hydrous magnesium silicate. The filtrate is concentrated while adding hexane to separate crystals. The material is recrystallized from ethyl acetate to give 11.68 g. of a light yellow crystalline material. The material is dried *in vacuo* to give the product of the Example as yellow crystals, m.p. 142-147°C.

Example 90

2-(3-Amino-4-methyl-2-pyrazolin-1-yl)-6-methylpyridine

A 200 ml. amount of hydrazine hydrate is added to 100 g. of 6-chloro-2-picoline (98%) and the mixture is refluxed for 16 hours.

The excess hydrazine is removed *in vacuo* and the residual oil is dissolved in dichloromethane and extracted with three 100 ml. portions of water. The organic layer is dried over anhydrous sodium sulfate and evaporated *in vacuo* to an oil. Evaporation is continued until crystals begin to form then hexane is added to separate a crystalline mass. This material is recrystallized from hexane after treatment with activated charcoal to yield 26.57 g. of 2-hydrazino-6-methylpyridine, m.p. 53-56°C.

A 0.51 g. amount of sodium metal is dissolved in 150 ml. of absolute ethanol, then 12.32 g. of 2-hydrazino--6-methylpyridine is added, followed by 6.7 g. of methacrylonitrile. The reaction mixture is refluxed on a steam

bath for 16 hours then is evaporated *in vacuo* to an oil. Water is added to separate a solid. The solid is collected and recrystallized from ethyl acetate after treatment with activated charcoal to give 10.12 g. of the desired product as white crystals, m.p. 181-185°C.

## Example 91

## 2-(3-Amino-4-methyl-2-pyrazolin-1-yl)pyridine

A 1.02 g. amount of sodium metal is dissolved in 250 ml. of absolute ethanol, then 21.8 g. of 2-hydrazinopyridine is added, followed by 13.4 g. of methacrylonitrile. The reaction mixture is refluxed for 16 hours, then the solvent is removed *in vacuo*. The solid is recrystallized twice from 3A ethanol after treatment with activated charcoal to give 4.30 g. of a tan solid. The above recrystallization filtrates are combined and evaporated *in vacuo* to a solid. The solid is dissolved in dichloromethane and passed through a hydrous magnesium silicate. The filtrate is concentrated while adding hexane at reflux to crystallize an additional 16.4 g. of the desired product (total 20.79 g.) as tan crystals, m.p. 149-151°C.

Example 92

2-(3-Amino-5-phenyl-2-pyrazolin-1-yl)pyridine

A 1.02 g. amount of sodium metal is dissolved in 250 ml. of absolute ethanol, then 21.82 g. of 2-hydrazinopyridine is added, followed by 25.8 g. of cinnamonitrile. The reaction mixture is refluxed for 16 hours, then the solvent is removed in vacuo. Water is added and the solid is collected by filtration. The solid is recrystallized from 3A ethanol to yield 20.32 g. of the desired product as yellow crystals, m.p. 203-206°C.

Example 93

N-[1-(6-Chloro-2-pyridyl)-4-methyl-2-pyrazolin-3-yl]acetamide

A mixture of 100 g. of 2,6-dichloropyridine in 2 ml. of hydrazine hydrate is stirred and refluxed for 5 hours. The reaction mixture is cooled and filtered. The product is washed with water and dried to give 47.7 g. of 2-chloro-6-hydrazinopyridine, m.p. 117-119°C.

A 500 mg. amount of sodium metal is dissolved in 100 ml. of absolute ethanol, then 15.0 g. of 2-chloro-6-hydrazinopyridine is added followed by 7.2 g. of methacrylonitrile. The reaction mixture is refluxed for 18 hours. The solvent is removed in vacuo and water is added to the residue to separate a solid. The solid is collected by filtration, dissolved in dichloromethane, dried over magnesium sulfate, and filtered through a hydrous magnesium silicate. The effluent is concentrated while adding hexane to crystallize 15.0 g. of 2-(3-amino-4-methyl-2-pyrazolin-1-yl)-6-chloropyridine as a tan solid, m.p. 170-175°C.

A mixture of 10.0 g. of the preceding compound, 500 mg. of 4-dimethylaminopyridine and 50.0 ml. of acetic anhydride is stirred for 10 minutes at room temperature to achieve solution. The solution is allowed to stand at room temperature for 18 hours to separate a solid. The whole mixture is poured into water and after standing one hour the solid is collected by filtration. The solid is

dissolved in dichloromethane. This solution is washed with water then dried over magnesium sulfate and evaporated *in vacuo* to give a solid. The solid is dissolved in 100 ml. of methanol and 10.0 ml. of 1N potassium hydroxide in methanol is added. After one hour at room temperature the organic solvent is removed *in vacuo* and water is added to separate a solid. The solid is collected, washed with water and dried to give 8.7 g. of product. A 500 mg. amount is recrystallized from dichloromethane-hexane to give 408 mg. of the prpoduct of the Example as pink crystals, m.p. 166-167°C.

Example 94

N-[1-(6-Chloro-2-pyridyl)-5-ethyl-2-pyrazolin--3-yl]acetamide

A solution of 124 g. of sodium methylate in 414 g. of methanol, diluted with 51 ml. of N,N-dimethylformamide is added via a dropping funnel to a stirred solution of 100 g. of diethylcyanomethylphosphorate and 35 g. of freshly distilled propionaldehyde in 75 ml. of N,N-dimethylformamide maintained at 40-45°C. in an ice bath. After the addition is completed the reaction mixture is warmed to 50°C. and stirring is continued for one hour without further external heating or cooling. The reaction mixture is diluted with 270ml. of50:50 methanol-water then the pH of the mixture is adjusted to pH 7.0 with glacial acetic acid and the neutral solution is extracted thoroughly with ether. The combined ether extracts are washed with dilute acetic acid then, with water. The organic layer is dried over magnesium sulfate, filtered and concentrated *in vacuo* to an oil. The oil is distilled through a 2 foot saddle filled column, giving 29.0 g. of 3-methoxyvaleronitrile, b.p. 42°C. at 3 mm., $n_D^{25°}$ 1.4190.

A 500 mg. amount of sodium metal is dissolved in 100 ml. of absolute ethanol, then 15.0 g. of 2-chloro-6-hydrazinopyridine (Example 93) is added followed by 11.0 g. of 3-methoxyvaleronitrile. The reaction mixture

is refluxed for 18 hours, then is evaporated in vacuo to give a solid. The solid is dissolved in water/dichloromethane. The organic layer is separated, dried over magnesium sulfate, filtered through hydrous magnesium silicate and concentrated while adding hexane to yield 14.3 g. of 2-(3-amino-5-ethyl-2-pyrazolin-1-yl)-6-chloropyridine as light yellow crystals, m.p. 125-127°C.

A mixture of 9.3 g. of the preceding compound, 500 mg. of 4-dimethylaminopyridine and 50.0 ml. of acetic anhydride is stirred until the solid gradually dissolves. The solution is allowed to remain at room temperature for 18 hours. The solution is then added to water to separate a solid. After standing for one hour the solid is collected by filtration and dissolved in 50 ml. of methanol, then 10 ml. of 1N potassium hydroxide in methanol is added. After standing at room temperature for 30 minutes this mixture is added to water to separate a solid. The solid is collected and dried to give 6.9 g. of crude product. A 300 mg. amount of this material is recrystallized from dichloromethane-hexane to yield 252 mg. of the desired product as white crystals, m.p. 170-171°C.

## Example 95

## N-[1-(6-Chloro-2-pyridyl)-2-pyrazolin-3-yl]-acetamide

A 0.32 g. amount of sodium metal is dissolved in 100 ml. of absolute ethanol, then 10.0 g. of 2-chloro-6-hydrazinopyridine (Example 93) is added, followed by 4.0 g. of acrylonitrile. The reaction mixture is refluxed for 18 hours. The solvent is removed in vacuo and water is added to the residue to separate a solid. The solid is collected by filtration, dissolved in dichloromethane, dried over magnesium sulfate, filtered through magnesium silicate and concentrated while adding hexane, to separate crystals. The mixture is cooled and filtered to give 9.3 g. of 2-(3-amino-2-pyrazolin-1-yl)-6-chloropyridine as a pale yellow solid, m.p. 143-145°C.

A mixture of 5.0 g. of the above compound, 50 mg. of 4-dimethylamino pyridine and 25.0 ml. of acetic anhydride is allowed to stand at room temperature for 16 hours. The solid formed is collected by filtration, washed with cold acetic anhydride then with hexane to give 2.25 g. of crude product. This material is dissolved in acetone. The solution is passed through a hydrous magnesium silicate and the filtrate is concentrated while adding hexane to crystallize 1.62 g. of the desired product as colorless needles, m.p. 209-211.5°C.

Example 96

N-[1-(6-Chloro-2-pyridyl)-2-pyrazolin-3-yl]-formamide

A mixture of 5.0 g. of 2-(3-amino-2-pyrazolin-1-yl)-6-chloropyridine (prepared as described in Example 95) and 25 ml. of a mixture of formic acid and acetic anhydride (Feiser and Feiser, Reagents for Organic Synthesis, Vol. 1, page 4) at room tempeature gives an immediate yellow precipitate. The precipitate is collected by filtration then is recrystallized from acetone-hexane to give 3.05 g. of the product of the Example as off-white crystals, m.p. 190-192°C.

Example 97

{{[1-(p-Chlorophenyl)-2-pyrazolin-3-yl]amino}-methylene}-malonic acid, diethyl ester

A) A 2.8 g. amount of sodium metal is dissolved in 200 ml. of absolute ethanol, then 17.9 g. of p-chlorophenylhydrazine hydrochloride is added followed in 25 minutes by 5.5 g. of acrylonitrile. The reaction mixture is refluxed for 6 hours and filtered while hot. The filtrate is evaporated to dryness then water is added to separate a solid. The solid is collected by filtration and dissolved in dichloromethane. This solution is passed through a short column of a hydrous magnesium silicate. The effluent is heated to boiling and hexane is added to crystallize a product. The mixture is cooled and filtered to give 8.75 g. of 3-amino-1-(p-chlorophenyl)-2-pyrazoline

as colorless needles, m.p. 142.5-145°C.

B) A mixture of 3.90 g. of the preceding product and 4.32 g. of ethoxymethylenediethylmalonate is heated at reflux for 2 hours then the solution is evaporated to dryness to give a solid. The solid is dissolved in methylene chloride and treated with a hydrous magnesium silicate and hexane as previously described to collect a solid. The solid is recrystallized from acetone-hexane to give 4.78 g. of the product of the Example as yellow prisms, m.p. 159-162°C.

Example 98

1-(p-Chlorophenyl)-5-methyl-3-methylamino-
-2-pyrazoline

A) A 1.72 g. amount of sodium metal is dissolved in 110 ml. of absolute ethanol, then 11.0 g. of p-chlorophenylhydrazine hydrochloride is added followed in 15 minutes by 4.20 g. of crotononitrile. The reaction mixture is refluxed for 18 hours. The solvent is removed *in vacuo* and water is added to the residue to separate a gum. The gum is dissolved in dichloromethane and separated from the aqueous layer. The organic layer is dried over anhydrous magnesium sulfate and filtered through a short column of a hydrous magensium silicate. The effluent is evaporated to give a glass. A small amount of benzene and then hexane is added to the glass which gradually solidifies. The solid is collected, washed with hexane and dried to give 8.1 g. of 3-amino-1-(p-chlorophenyl)-5-methyl-2-pyrazoline as a pink solid, m.p. 90-92°C.

B) A 4.8 g. amount of the preceding compound is dissolved in 25 ml. of a mixture of formic acid and acetic anhydride (Feiser and Feiser, Reagents for Organic Synthesis, Vol. 1, page 4) and allowed to remain at room temperature for 3 hours. Then ice water is added to the mixture to give a gum. The aquecus phase is decanted and the gum is dissolved in dichloromethane. The solution is dried over magnesium sulfate, filtered through a hydrous

magnesium silicate and evaporated leaving a yellow gum. This material is dissolved in dichloromethane and heated to boiling, then hexane is added until turbidity occurs. The mixture is cooled and filtered to yield 3.6 g. of N--[1-(p-chlorophenyl)-5-methyl-2-pyrazolin-3-yl]formamide as off-white crystals, m.p. 112-113°C.

C) To a stirred solution of 250 ml. of freshly distilled tetrahydroufuran under nitrogen is added cautiously 3.3 g. of lithium aluminum hydride, then 3.3 g. of N-[1-(p-chlorophenyl)-5-methyl-2-pyrazolin-3-yl]formamide is added and the mixture is refluxed under nitrogen for 4 hours. The reaction mixture is cooled. Then with stirring, 3.3 ml. of water is cautiously added dropwise, followed by 3.3 ml. of 15% aqueous sodium hydroxide solution and 10.0 ml. of water. The mixture is filtered and the residue is washed with ether. The combined filtrate and wash is evaporated to dryness giving a gum which solidifies. The solid is collected, washed with hexane and dried. The product of the Example is recrystallized from dichloromethane-hexane to give 2.3 g. of white crystals, m.p. 102-103°C.

## Example 99

## 1-(3,4-Dichlorophenyl)-3-methylamino--2-pyrazoline

A) A 9.9 g. amount of sodium metal is dissolved in 450 ml. of absolute ethanol, then 75.0 g. of 3,4-dichlorophenylhydrazine hydrochloride is added, followed in 10 minutes by 19.5 g. of acrylonitrile. The reaction mixture is refluxed for 18 hours then the solvent is removed *in vacuo*. Water is added to the residue to separate a granular solid. The solid is collected by filtration and dissolved in acetone. This solution is filtered and the filtrate is evaporated to dryness. The resulting residue is triturated with ether to give 62.4 g. of 3--amino-1-(3,4-dichlorophenyl)-2-pyrazoline as a tan crystalline solid, m.p. 181-183°C.

B) A 10.0 g. amount of the preceding product is dissolved in 50 ml. of a mixture of formic acid and acetic anhydride (Feiser and Feiser, Reagents for Organic Synthesis, Vol. 1, page 4) and allowed to remain at room temperature for 2 hours. The resulting solid is collected by filtration and dissolved in dichloromethane. The organic solution is washed with water, dried over anhydrous magnesium sulfate and filtered through a short column of a hydrous magnesium silicate. The effluent is concentrated, adding hexane until turbidity occurs. The mixture is cooled and filtered to give 6.5 g. of N-[1-(3,4-dichlorophenyl)-3-pyrazolin-3-yl]formamide as a white solid, m.p. 148-149°C.

C) To a stirred solution of 250 ml. of freshly distilled tetrahydrofuran under nitrogen is added cautiously 5.0 g. of lithium aluminum hydride, then 5.0 g. of N-[1-(3,4-dichlorophenyl)-3-pyrazolin-3-yl)formamide is added portion-wise and the mixture is refluxed under nitrogen for 6 hours. The reaction mixture is cooled and the excess hydride is decomposed by the caution dropwise addition of 5.0 ml. of water followed by 5.0 ml. of 15% sodium hydroxide solution and 15 ml. of water. The mixture is filtered and the filtrate is evaporated to give a gum and solid. This material is dissolved in dichloromethane and passed through a short column of a hydrous magnesium silicate. The effluent is evaporated to give a dark gum and solid. The mixture is dissolved in ether and treated as above with a hydrous magnesium silicate. The effluent is evaporated and the solid is recrystallized twice from ether-hexane to give 2.0 g. of the desired product as white crystals, m.p. 68-70°C.

Example 100

1-(p-Chlorophenyl)-4-methyl-3-methylamino--2-pyrazoline

A) A 1.2 g. amount of sodium metal is dissolved in 110 ml. of absolute ethanol, then 7.5 g. of p-chlorophenylhydrazine hydrochloride is added followed by 2.9 g.

of methacrylonitrile. The reaction mixture is refluxed for 18 hours. The solvent is removed in vacuo and water is added to the residue to separate a gum. The gum is collected and dissolved in dichloromethane. The organic solution is washed with water, dried over anhydrous magnesium sulfate and filtered through a short column of a hydrous magnesium silicate. The effluent is evaporated leaving a gum which solidifies upon adding hexane. The solid is collected and recrystallized from ether-hexane to give 4.6 g. of 3-amino-1-(p-chlorophenyl)-4-methyl-2--pyrazoline as white crystals, m.p. 107-108°C.

B) A 2.2 g. amount of the preceding product is dissolved in 10.0 ml. of a mixture of formic acid and acetic anhydride [Example 99 (B) ]. The mixture is allowed to remain at room temperature for 3 hours. Water is added and a yellow solid is collected by filtration. The solid is dissolved in dichloromethane. The solution is dried, filtered and concentrated as described in Example 99 (B) to give 2.1 g. of N-[1-(p-chlorophenyl)-4--methyl-2-pyrazolin-3-yl]formamide as a white solid, m.p. 172-174°C.

C) A 1.9 g. amount of the preceding product is added to a suspension of 1.9 g. of lithium aluminum hydride in 100 ml. of freshly distilled tetrahydrofuran under nitrogen and refluxed for 4 hours as described in Example 99 (C). The reaction mixture is cooled and the excess hydride is decomposed as previously described with water and 15% sodium hydroxide. The mixture is filtered and the filtrate is evaporated. The residue is dissolved in dichloromethane and the solution is passed through a short column of a hydrous magnesium silicate. The effluent is evaporated in vacuo to give a cloudy gum. The gum is dissolved in ether and filtered. The filtrate is evaporated in vacuo and cooled. The addition of hexane provides crystals. The crystals are collected and dissolved in ether. The solution is filtered then hexane is added until turbidity appears. The solution is cooled and

and seeded to yield 1.18 g. of the product of the Example as white crystals, m.p. 70-72°C.

Example 101

1-(m-Chlorophenyl)-4-methyl-3-methylamino--2-pyrazoline

A) A 3.12 g. amount of sodium metal is dissolved in 200 ml. of absolute ethanol, then 20.9 g. of m--chlorophenylhydrazine hydrochloride is added, followed by 7.6 g. of methacrylonitrile. The reaction mixture is refluxed, extracted and filtered as for Example 100 (A) to give a solid. The solid is recrystallized twice from ether-hexane to give 16.2 g. of 3-amino-1-(m-chlorophenyl)-4-methyl-2-pyrazoline as white crystal, m.p. 84-85°C.

B) A 3.0 g. amount of the preceding product is dissolved in 10 ml. of a mixture of formic acid and acetic anhydride [Example 99 (B)] and is allowed to remain at room temperature for 3 hours. The reaction mixture is then poured into water to separate a partial gum and solid. This material is collected, dissolved in dichloromethane and filtered through a short column of a hydrous magnesium silicate. The effluent is concentrated while adding hexane to separate 3.1 g. of N-[1-(m-chlorophenyl)--4-methyl-2-pyrazolin-3-yl]formamide as light yellow crystals, m.p. 140-142°C.

C) As for Example 99 (C), a 2.0 g. amount of the preceding compound is added to a suspension of 2.0 g. of lithium aluminum hydride in 200 ml. of freshyl distilled tetrahydrofuran under nitrogen and refluxed for 4 hours. The reaction mixture is cooled and the excess hydride is decomposed and treated as described in Example 99 (C) to give a solid. The solid is recrystallized from ether-hexane to give 1.3 g. of crude product. A 200 mg. amount of this material is recrystallized twice more from ether-hexane after filtration through diatomaceous earth to give 100 mg. of the desired product as white crystals, m.p. 75-76°C.

## Example 102

## 1-(p-Chlorophenyl)-5-methyl-3-(2,2,2-trifluoroethylamino)-2-pyrazoline

A) A 8.0 g. amount of 3-amino-1-(p-chlorophenyl)-5-methyl-2-pyrazoline [prepared as described in Example 98 (A)] is dissolved in 25.0 ml. of dichloromethane with stirring. This solution is slowly added with stirring to 25.0 ml. of trifluoroacetic anhydride cooled at 10°C. The reaction mixture is stirred for 3 hours at room temperature then the solvent is removed *in vacuo*. The residue is dissolved in dichloromethane and this solution is passed through a short column of a hydrous magnesium silicate. The effluent is concentrated while adding hexane until turbidity results. The solution is cooled, then filtered to give 8.5 g. of N-[1-(p-chlorophenyl)-5-methyl-2-pyrazolin-3-yl]-2,2,2-trifluoroacetamide as a white solid, m.p. 170-172°C.

B) To a stirred solution of 250 ml. of freshly distilled tetrahydrofuran under nitrogen is added cautiously 5.0 g. of lithium aluminum hydride, then 5.0 g. of the above product is added portionwise to the mixture. The reaction mixture is stirred under nitrogen and refluxed for 7 hours. The reaction mixture is cooled and the excess hydride is decomposed by the cautious dropwise addition of 5.0 ml. of water followed by 5.0 ml. of 15% sodium hydroxide solution and 15 ml. of water. The mixture is filtered and the filtrate is evaporated leaving a thin oil. The oil is dissolved in dichloromethane. This solution is dried over anhydrous magnesium sulfate, passed through a hydrous magnesium silicate and evaporated to give a gum which solidifies when triturated with hexane. The solid is recrystallized from dichloromethan--hexane to give 2.8 g. of the product of the Example as white crystals, m.p. 63-64°C.

Example 103

1-(p-Chlorophenyl)-4-methyl-3-[(2,2,2-trifluoroethyl)amino]-2-pyrazoline

A) A 7.0 g. amount of 3-amino-1-(p-chlorophenyl)-4-methyl-2-pyrazoline [prepared as described in Example 100 (A)] is dissolved in 25.0 ml. of dichloromethane and cooled to 5°C. The solution is stirred and 14.0 ml. of trifluoroacetic anhydride is added. Stirring is continued at room temperature for 3 hours, then the solvent is removed *in vacuo*. The residue is dissolved in dichloromethane and the solution is concentrated with the addition of hexane until turbidity appears. The mixture is cooled and the solid is collected to give 5.3 g. of product. A 1.0 g. amount of this material is recrystallized from dichloromethane-hexane to give 750 mg. of N-[1-(p-chlorophenyl)-4-methyl-2-pyrazolin-3-yl]-2,2,2--trifluoroacetamide as a white solid, m.p. 181-183°C.

B) To a stirred solution of 400 ml. of freshly distilled tetrahydrofuran under nitrogen is added cautiously 6.9 g. of lithium aluminum hydride, then 6.9 g. of the preceding product (prepared as described above) is added portionwise to the mixture. The reaction mixture is stirred under nitrogen and refluxed for 5 hours. The reaction mixture is cooled and the excess hydride is decomposed by the cautious dropwise addition of 6.9 ml. of water followed by 6.9 ml. of 15% sodium hydroxide solution and 20.7 ml. of water. The mixture is filtered and the filtrate is evaporated leaving a crude tacky tan solid. The solid is dissolved in ether and filtered through a micronized synthetic magnesium silicate adsorbent. The effluent is concentrated while adding hexane until turbidity occurs. The mixture is cooled in a refrigerator then filtered to give 3.4 g. of the desired product as a white solid, m.p. 84-86°C.

Example 104

1-(p-Chlorophenyl)-3-ethylamino-5-methyl-2--pyrazoline

A mixture of 8.0 g. of 3-amino-1-(p-chlorophenyl)-5-methyl-2-pyrazoline [prepared as described in Example 98 (A)], 400 mg. of 4-dimethylamine pyridine and 20.0 ml. of acetic anhydride is allowed to stand at room temperature for 48 hours. The solid formed is collected by filtration, washed with cold acetic anhydride then with hexane and is dried to give a white solid. The solid is recrystallized from dichloromethane-hexane to give 6.6 g. of N-[1-(p-chlorophenyl)-5-methyl-2-pyrazolin-3-yl]acetamide as a white solid, m.p. 167-169°C.

To a stirred solution of 300 ml. of freshly distilled tetrahydrofuran under nitrogen is added cautiously 6.0 g. of lithium aluminum hydride, then 6.0 g. of the preceding product is added portionwise to the mixture. The reaction mixture is stirred under nitrogen and refluxed for 5 hours. The reaction mixture is cooled and the excess hydride is decomposed as described in Example 103(B) using 6.0 ml. of water, 6.0 ml. of 15% sodium hydroxide solution and 18.0 ml. of water. The residual solid is filtered and washed with tetrahydrofuran. The combined filtrate and wash is evaporated to give a thin oil. The oil is dissolved in dichloromethane, filtered through a hydrous magnesuum silicate and evaporated to give a thin oil. The addition of hexane separates 4.7 g. of crystals. A 200 mg. amount of the solid is recrystallized from ether-hexane to give 138 mg. of the desired product as a white solid, m.p. 57-58°C.

Example 105

1-(p-Chlorophenyl)-3-ethylamino-4-methyl-2-pyrazoline

A) A 1.2 g. amount of sodium metal is dissolved in 110 ml. of absolute ethanol, then 7.5 g. of p-chlorophenylhydrazine hydrochloride is added followed by 2.9 g. of methacrylonitrile. The reaction mixture is refluxed for 18 hours. The procedure of Example 98(A) is followed

through the column filtration step. The effluent is evaporated leaving a gum which solidifies upon adding hexane. The solid is recrystallized from ether-hexane to give 4.6 g. of 3-amino-1-(p-chlorophenyl)-4-methyl-2--pyrazoline as white crystals, m.p. 107-108°C.

B) A 9.0 g. amount of the preceding compound (prepared as described above) is dissolved in 54.0 ml. of acetic anhydride. The warm solution is allowed to stand for 16 hours at room temperature with some solid separation. Water is added to complete the solid separation. The solid is collected by filtration and is dissolved in methanol. Then 1N potassium hydroxide in methanol is added to make basic. After 15 minutes at room temperature the solvent is removed in vacuo and water is added to separate a solid. The solid is collected and recyrstallized from dichloromethane-hexane to give 6.5 g. of N--[1-(p-chlorophenyl)-4-methyl-2-pyrazolin-3-yl]acetamide as white crystals, m.p. 172-173°C.

C) A 6.5 g. amount of the above compound is added to a stirred suspension under nitrogen of 6.5 g. of lithium aluminum hydride in 250 ml. of freshly distilled tetrahydrofuran. The stirred reaction mixture is refluxed for 5 hours under nitrogen. Then the mixture is cooled and the excess hydride is decomposed as described in Example 103 using 6.5 ml. of water, 6.5 ml. of 15% sodium hydroxide solution and 13 ml. of water. The residual solid is filtered and washed with tetrahydrofuran. The combined filtrate and wash is evaporated to a thin oil which crystallizes. The solid is collected and recrystallized from dichloromethane-hexane to give 4.6 g. of product. A 115 mg. amount of product is recrystallized from ether-hexane to give 90 mg. of the desired product as white crystals, m.p. 63-64°C.

Example 106

1-(3,4-Dichlorophenyl)-3-(2,2,2-trifluoroethylamino)--2-pyrazoline

A 10.0 g. amount of 3-amino-1-(3,4-dichlorophenyl)-2-pyrazoline [Example 99 (A)] is added portionwise with cooling and stirring to 50 ml. of trifluoroacetic anhydride with separation of a solid. The reaction mixture is stirred for 3 hours at room temperature then the solid is collected by filtration and dried to give 12.2 g. of product. A 3.0 g. amount of this material is recrystallized from dichloromethane-hexane to give 2.4 g. of N-[1-(3,4-dichlorophenyl)-2-pyrazolin-3-yl]-2,2,2-trifluoroacetamide as pale yellow needles, m.p. 163--164°C.

A 5.0 g. portion of the above product is refluxed for 4 hours with lithium aluminum hydride in tetrahydrofuran as described in Example 102 (B). The hydride is decomposed as described and the reaction mixture is filtered. The filtrate is evaporated to give a dark gum. The gum is dissolved in dichloromethane and filtered through a hydrous magnesium silicate. The filtrate is evaporated to give a thin green oil which crystallizes on standing. This material is recrystallized twice with hexane to give 3.6 g. of the desired product as a grey solid, m.p. 93-94°C.

Example 107

3-Ethylamino-1-(*m*-fluorophenyl)-4-methyl--2-pyrazoline

A) A 2.8 g. amount of sodium metal is dissolved in 200 ml. of absolute ethanol, then 16.2 of *m*--fluorophenylhydrazine hydrochloride is added followed by 13.4 g. of methacrylonitrile. The reaction mixture is refluxed for 18 hours then the solvent is evaporated *in vacuo*. Water is added to give a gum. The gum is dissolved in dichloromethane and the solution is passed through a short column of a hydrous magnesium silicate. The effluent is concentrated while adding hexane until

turbidity occurs. The solution is cooled and seeed to separate a pink solid which is collected by filtration and dried to give 4.2 g. of 3-amino-1-(m-fluorophenyl)-4--methyl-2-pyrazoline.

B) A 4.2 g. amount of the preceding compound is dissolved in 10.0 ml. of acetic anhydride then 100 mg. of 4-dimethylamino pyridine is added and the reaction mixture is allowed to stand at room temperature for 16 hours. The mixture is poured into water to separate a gum which solidifies. The solid is collected by filtration and washed with water. The solid is dissolved in 50.0 ml. of methanol and 10.0 ml. of 1N sodium hydroxide in methanol is added. The mixture is allowed to stand at room temperature for 30 minutes then the solvent is partly removed in vacuo. The addition of water separates a yellow solid. The solid is collected and dried to give 4.1 g. of N-[1-(m-fluorophenyl)-4-methyl-2-pyrazolin-3-yl]-acetamide, m.p. 171-172°C.

C) A 4.5 g. amount of the preceding compound (prepared as described above) is added to a stirred suspension under nitrogen of 4.5 g. of lithium aluminum hydride in 250 ml. of freshly distilled tetrahydrofuran. The reaction mixture is stirred and refluxed for 5 hours under nitrogen. The mixture is cooled and the excess hydride is decomposed as described in Example 103(B) using 4.5 ml. of water, 4.5 ml. of 15% sodium hydroxide solution and 14 ml. of water. The mixture is filtered and the filtrate is evaporaed in vacuo to give a gum. The gum is placed on a column containing 80 g. of a synthetic magnesium silicate adsorbent. The column is eluted first with hexane (cuts 1 and 2), then with 2% acetone-hexane (cuts 3 and 4 combined and cuts 5, 6 and 7 combined), then with 4% acetone-hexane (cuts 8, 9 and 10 combined). The elution procedure is monitored by thin-layer chromatography using the upper phase of a mixture of 2 parts benzene, 1 part acetone and 2 parts water to develop the plate. The fraction represented by combining cuts 5, 6 and 7 is evaporated to give 0.9 g. of the desired product as a pink oil.

Example 108

3-Ethylamino-4-methyl-1-phenyl-2-pyrazoline

A 2.0 g. amount of sodium metal is dissolved in 200 ml. of absolute ethanol, then 37.4 g. of phenylhydrazine is added followed in 10 minutes by 20.1 g. of methacrylonitrile. The reaction mixture is relfuxed for 4 hours then is evaporated to near dryness in vacuo. Water is added to the residue to separate an oil. The oil crystallizes on standing. The solid is dissolves in dichloromethane. The solution is passed through a short column of a hydrous magnesium silicate. The column effluent is evaporated to give an oil. The oil is crystallized from acetone-hexane then is recrystallized from the same solvent pair to give 20.88 g. of 3-amino-4-methyl-1-phenyl-2-pyrazoline as colorless crystals, mp. 83-84°C.

A 10.0 g. amount of the preceding product is dissolved in 50 ml. of acetic anhydride, then 500 mg. of 4-dimethylaminopyridine is added and the reaction mixture is allowed to stand at room temperature for 18 hours. The mixture is poured into water to separate a gum which solidifies and is collected by filtration. The solid is added with stirring to 150 ml. of methanol containing 10.0 g. of sodium hydroxide. After 30 minutes the solvent is removed in vacuo and water is added to provide a gum which becomes solid. This solid is dissolved in dichloromethane, dried over anhydrous magnesium sulfate and passed through a short column of a hydrous magesium silicate. The effluent is concentrated while adding hexane to yield crystals. The material is collected and dried to give 3.3 g. of N-(4--methyl-1-phenyl-2-pyrazolin-3-yl)acetamide as a white solid, m.p. 149-150°C.

A 6.0 g. amount of the preceding compound (prepared as described above) is added to stirred suspension under nitrogen of 6.0 g. of lithium aluminum hydride in 250 ml. of freshly distilled tetrahydrofuran. The reaction mixture is stirred and refluxed for 5 hours under nitrogen.

The mixture is cooled and the excess hydride is decomposed as described in Example 103(B) using 6.0 ml. of water, 6.0 ml. of 15% sodium hydroxide solution and 18 ml. of water, 6.0 ml. of 15% sodium hydroxide solution and 18 ml. of water. The mixture is filtered and the filtrate is evaporated <u>in vacuo</u> to give an oil. The oil is placed on a column containing 80 g. of a 200 mesh synthetic magnesium silicate adsorbent. The column is eluted first with 2% acetone hexane (cut 1), then with 4% acetone-hexane (cuts 2, 3, 4, 5 and 6) and finally with 8% acetone-hexane (cut 7). The elution procedure is monitored by thin layer chromatography using the system described in Example 107(C). The fraction represented by combining cuts 2, 3, 4, 5 and 6 is evaporated to give 4.3 g. of the desired product as a pink oil.

## Example 109

### N-[1-(3,4-Dichlorophenyl)-2-pyrazolin-3-yl]-2,2,2-trifluoroacetamide

A 9.9 g. amount of sodium metal is dissolved in 450 ml. of absolute ethanol, then 75.0 g. of 3,4-dichlorophenylhydrazine hydrochloride is added followed in 10 minutes by 19.5 g. of acrylonitrile. The reaction mixture is refluxed for 18 hours. The solvent is removed <u>in vacuo</u>. Water is added to separate a granular solid. The solid is dissolved in acetone and filtered to remove a small amount of insoluble material. The filtrate is evaporated to dryness leaving a solid. The solid is triturated with ether to give 62.4 g. of 3-amino-1-(3,4-dichlorophenyl)-2-pyrazoline as a tan crystalline solid, m.p. 181-183°C.

A 10.0 g. amount of the preceding product is added portionwise with cooling and stirring to 50 ml. of trifluoroacetic anhydride with separation of a solid. The reaction mixture is stirred for 3 hours at room temperature then the solid is collected by filtration and dried to give 12.2 g. of product. A 3.0 g. amount of this material is recrystallized from dichloromethane-hexane to give 2.4 g. of the product of the Example as pale yellow needles, m.p.

163-164°C.

Example 110

p-Chloro-N-[1-(3,4-dichlorophenyl)-2--pyrazolin-3-yl]benzamide

A stirred mixture of 2.30 g. of 3-amino-1-(3,4--dichlorophenyl)-2-pyrazoline (prepared as described in Example 109) in 20 ml. of dry pyridine is cooled in an ice bath, then 2.10 g. of p-chlorobenzoyl chloride is added. The reaction mixture is allowed to stand at room temperature for 16 hours then is poured into water to separate a solid. The solid is collected by filtration and dissolved in dichloromethane. This solution is passed through a short column of a hydrous magnesium silicate. The effluent is refluxed and hexane is added until turbidity results. The solution is cooled and filtered to collect a product. The product is recrystallized from the same solvent pair to give 1.8 g. of the desired product as yellow needles, m.p. 177-178°C.

Example 111

2,2,2-Trifluoro-N-[5-p-tolyl-1-(p-trifluoroacetylphenyl)-2-pyrazolin-3-yl]acetamide

A 1.0 g. amount of sodium metal is dissolved in 100 ml. of absolute ethanol, then 20.0 ml. of phenylhydrazine is added followed in 5 minutes by 24.0 ml. of (mixed cis and trans) 4-methylcinnamonitrile. The reaction mixture is refluxed for 4 hours then is cooled. The precipitate is collected by filtration then is recrystallized from acetone-hexane after treatment with activated charcoal to give 14.25 g. of 3-amino-1-phenyl--5-p-tolyl-2-pyrazoline as pale orange needles, m.p. 165--166°C.

A mixture of 5.0 g. of the preceding product and 25 ml. of trifluoroacetic anhydride is allowed to stand at room temperature for 5 minutes. The mixture is filtered and the solid collected is dissolved in dichloromethane, then the solutionis evaporated to dryness.

The residue is again dissolved in dichloromethane and is passed through a hydrous magnesium silicate and recrystallized as described in Example 110 to give 1.5 g. of the product of the Example as matted pale yellow needles, m.p. 206-207°C.

Example 112

2,2,2-Trifluoro-N-[5-phenyl-1-(p-trifluoroacetyl phenyl)-2-pyrazolin-3-yl]acetamide

A 2.0 g. amount of sodium metal is dissolved in 150 ml. of absolute ethanol, then 40.0 ml. of phenylhydrazine is added followed in 5 minutes by 48.0 ml. of cinnamonitrile. The reaction mixture is refluxed for 3 hours with exothermic crystallization of a product. The product is collected by filtration and washed with water. The material is recrystallized from absolute ethanol to give 56.0 g. of 3-amino-1,5-diphenyl-2-pyrazoline as a solid, m.p. 195-197°C.

A mixture of 5.0 g. of the above product and 25.0 ml. of trifluoroacetic anhydride is allowed to stand at room temperature for 16 hours. The solvent is removed *in vacuo* and the residue is taken up in dichloromethane. The solution is passed through a short column of a hydrous magnesium silicate and the product is recrystallized from the effluent as described in Example 110. The entire recrystallization procedure is repeated to give 3.0 g. of the desired product as yellow needles, m.p. 197-198°C.

Example 113

2,2,2-Trifluoro-N-[5-methyl-1-(p-trifluoroacetylphenyl)-2-pyrazolin-3-yl]acetamide

A) A 2.0 g. amount of sodium metal is dissolved in 200 ml. of absolute ethanol, then 32.4 g. of phenylhydrazine in 50 ml. of ethanol is added, followed in 10 minutes by 20.1 g. of crotononitrile. The reaction mixture is refluxed for 5 hours. Most of the ethanol is removed *in vacuo*, water is added and the product is collected by filtration. The solid is dissolved in dichloromethane. This solution is passed through a short column of a hydrous magnesium silicate. The column effluent is then refluxed

on a steam bath with the gradual addition of hexane to crystallize a product. The product is collected and recrystallized from acetone-hexane to give 26.9 g. of 3-amino-5-methyl-1-phenyl-2-pyrazoline as colorless prisms, m.p. 103.5-106°C.

B) A mixture of 5.0 g. of the preceding compound and 25.0 ml. of trifluoroacetic anhydride is allowed to stand at room temperature for 48 hours. Dichloromethane is added to dissolve the residual sludge. The solution is evaprated to dryness *in vacuo*. The residue is dissovled in dichloromethane. The solution is passed through a short column of a hydrous magnesium silicate. Hexane is added to the effluent to separate a green oily precipitate. The solvent is decanted and evaporated to give a solid. The solid is again dissolved in dichloromethane, column treated as above and recrystallized by the addition of hexane. The product is collected by filtration and the above procedure is repeated to give 1.15 g. of the product of the Example as yellow matted needles, m.p. 189-190.5°C.

## Example 114

## 2,2,2-Trifluoro-N-(4-methyl-1-phenyl-2--pyrazolin-3-yl)acetamide

A 2.0 g. amount of sodium metal is dissolved in 200 ml. of absolute ethanol, then 37.4 g. of phenylhydrazine is added followed in 10 minutes by 20.1 g. of methacrylonitrile. The reaction mixture is refluxed for 4 hours then is evaporated to near dryness *in vacuo*. Water is added to the residue to separate an oil. The oil is crystallized on standing. The solid is dissolved in dichloromethane and the solution is passed through a short column of a hydrous magnesium silicate. The column effluent is evaporated to give an oil. The oil is crystallized from acetone-hexane then is recrystallized from the same solvent pair to give 20.88 g. of 3-amino-4-methyl-1-phenyl-2-pyrazoline as colorless crystals m.p. 83-84°C.

A mixture of 5.0 g. of the preceding compound and 25.0 ml. of trifluoroacetic anhydride is allowed to stand for 2 minutes. The solid which separates is collected by filtration and washed with hexane. The solid is dissolved in dichloromethane. This solution is passed through a hydrous magnesium silicate and recrystallized as described in Example 110 to give 1.82 g. of the desired product as needles, m.p. 142-143.5°C.

Example 115

2,2,2-Trifluoro-N-[4-methyl-1-(p-trifluoroacetylphenyl)-2-pyrazolin-3-yl]acetamide

A mixture of 5.0 g. of 3-amino-4-methyl-1-phenyl-2-pyrazoline (prepared as described in Example 114) and 25.0 ml. of trifluoroacetic anhydride is allowed to stand at room temperature for 24 hours. Dichloromethane is added to dissolve the solid formed then the solvent is evaporated. The solid is again dissolved in dichloromethane. This solution is columnized and recrystallized as described in Example 110 to give 5.2 g. of the product of the Example as pale yellow needles, m.p. 203-204°C.

Example 116

2,2,2-Trifluoro-N-[1-(p-trifluoroacetylphenyl)-2-pyrazolin-3-yl]acetamide

A 2.0 g. amount of sodium metal is dissolved in 100 ml. of absolute ethanol, then 40.0 ml. of phenylhydrazine is added followed by 26.0 ml. of acrylonitrile. The reaction mixture is refluxed for 3 hours with exothermic crystallization of a product. The product is collected by filtration and washed with 95% ethanol. The material is recrystallized from dichloromethane-benzene to give 43.6 g. of 3-amino-1-phenyl-2-pyrazoline as a solid, m.p. 168-170.5°C.

A mixture of 1.5 g. of the preceding product and 8.0 ml. of trifluoroacetic anhydride (exothermic reaction) is allowed to stand at room temperature for one hour. The

mixture is evaporated to dryness in vacuo then dichloromethane is added. The solution is filtered and the filtrate is evaporated to give 1.2 g. of the desired product as yellow needles, m.p. 228.5-230.5°C.

Example 117

N-(1,5-Diphenyl-2-pyrazolin-3-yl)-2,2,2-trifluoroacetamide

A 5.0 g. amount of 3-amino-1,5-diphenyl-2-pyrazoline (prepared in Example 112) and 25.0 ml. of trifluoroacetic anhydride is heated until solution. After standing for 2 minutes, the precipitate formed is collected by filtration and washed with hexane. The solid is dissolved in dichloromethane and passed through a short column of a hydrous magnesium silicate. The effluent is refluxed and hexane is added to crystallize the product. The product is recrystallized from hexane to give 0.95 g. of the desired product as needles, m.p. 151.5-153.5°C.

Example 118

N-[1-(m-Chlorophenyl)-2-pyrazolin-3-yl]-2,2,2-trifluoroacetamide

A) A 1.0 g. amount of sodium metal is dissolved in 100 ml. of absolute ethanol, then 30.75 g. of m-chlorophenylhydrazine is added followed in 5 minutes by 12.0 g. of acrylonitrile. The reaction mixture is refluxed for 5 hours thenis cooled and filtered. The solid collected is dissolved in acetone, treated with activated charcoal and filtered. Hexane is added to the filtrate to crystallize the product which is collected to give 30.7 g. of 3-amino-1-(m-chlorophenyl)-2-pyrazoline as off-white prisms, m.p. 131-132°C.

B) A 5.0 g. amount of the above product is added to 25.0 ml. of trifluoroacetic anhydride giving an exothermic reaction with formation of a precipitate. After 5 minutes standing, the precipitate is collected by filtration. The solid is dissolved in dichloromethane then is passed through a short column of a hydrous magnesium silicate. The effluent is refluxed and hexane is added

until turbidity results. Then the mixture is cooled and filtered to provide 4.7 g. of the desired product as yellow crystals, m.p. 143-144°C.

Example 119

N-[1,5-Bis(p-chlorophenyl)-2-pyrazolin-3-yl]-2,2,2-trifluoroacetamide

Sodium metal (2.8 g.) is dissolved in 125 ml. of absolute ethanol, then 17.9 g. of p-chlorophenylhydrazine hydrochloride is added followed by 16.36 g. of p-chlorocinnamonitrile. The reaction mixture is refluxed for 7 hours and is allowed to stand at room temperature for 16 hours. The solvent is removed *in vacuo* and water is added to separate a solid. The solid is collected by filtration, dissolved in dichloromethane, and is columnized and crystallized as for Example 117. The product is recrystallized from acetone-hexane to give 10.0 g. of 3-amino-1,5-bis(p-chlorophenyl)-2-pyrazoline as colorless needles, m.p. 150-150.5°C.

A 5.0 g. amount of the preceding compound is mixed with 25.0 ml. of trifluoroacetic anhydride at room temperature. The mixture is allowed to stand for 16 hours, then is filtered. The solid is dissolved in dichloromethane. The solution is columnized and crystallized as described above to give 4.52 g. of the desired product as light yellow matted needles, m.p. 138.5-140°C.

Example 120

2-Bromo-N-[1-(α,α,α-trifluoro-m-tolyl)-2-pyrazolin-3-yl]propionamide

Sodium metal (2.8 g.) is dissolved in 125 ml. of absolute ethanol, then 21.2 g. of m-trifluoromethylphenylhydrazine hydrochloride is added followed by 5.5 g. of acrylonitrile. The reaction mixture is refluxed for 18 hours. The solvent is removed *in vacuo* and water is added to the residue to give a tacky solid. The solid is dissolved in dichloromethane. The solution is dried over anhydrous magnesium sulfate and passed through a short column of ahydrous magnesium silicate. The effluent is

collected and concentrated to separate tan crystals. This material is recrystallized from acetone-hexane to give 14.8 g. of 3-amino-1-(α,α,α-trifluoro-m-tolyl)-2-pyrazoline as pale yellow needles, m.p. 104-105°C.

To 100 ml. of chloroform with stirring is added 2.75 g. of potassium carbonate and 5.3 g. of 3-amino-1-(α,α,α-trifluoro-m-tolyl)-2-pyrazoline, then 5.0 g. of α-bromopropionylchloride is added dropwise. After the addition is complete the reaction mixture is refluxed on a steam bath for 4 hours. Then the solvent is removed *in vauco* and water is added to give a gum. The gum is extracted with dichloromethane. The extracts are combined and dried over anhydrous magnesium sulfate. The solution is passed through a short column of a hydrous magnesium silicate, then is evaporated leaving a gummy solid. The solid is triturated with ether to give 1.13 g. of a yellow solid. The solid is recrystallized from acetone-hexane to give the desired product as yellow crystals, m.p. 54-56°C.

## Example 121

## 2-Dimethylamino-N-[1-(α,α,α-trifluoro-m-tolyl)-2-pyrazolin-3-yl]propionamide

A 1.0 g. amount of 2-bromo-N-[1-(α,α,α-trifluoro-m-tolyl)-2-pyrazolin-3yl]propionamide (Example 120) is dissolved in 50 ml. of benzene in a pressue bottle, then 2.0 ml. of dimethylamine is added and the bottle is sealed for 1/2 hour at room temperature with the separation of crystals. The mixture is heated on a steam bath for 6 hours, then is cooled. The reaction mixture is partitioned between water and benzene. The benzene layer is dried over anhydrous magnesium sulfate and evaporated to a dark gum. The gum is dissolved in hexane and filtered through a hydrous magnesium silicate. The filtrate is evaporated to a gum (0.9 g.). A 210 mg. amount of this gum is chromatographed by thick layer chromatography on one mm Silica Gel G plates using the upper phase of a mixture of 2 parts benzene, 1 part acetone and 2 parts water to give 180 mg. of the desired product as a light yellow gum.

Example 122

N-[1-(3,4-Dichlorophenyl)-5-methyl-2-pyrazolin--3-yl]-2,2,2-trifluoroacetamide

A) A 2.8 g. amount of sodium metal is dissolved in 125 ml. of absolute ethanol, then 21.35 g. of 3,4-dichlorophenylhydrazine hydrochloride is added followed in 5 minutes by 6.7 g. of crotononitrile. The reaction mixture is refluxed for 8 hours, then is evaporated to dryness in vacuo. Water is added to separate an oil. The oil is crystallized, then is dissolved in dichloromethane and is passed through a short column of a hydrous magnesium silicate. The effluent is refluxed with the gradual addition of hexane to separate 14.4 g. of 3-amino-1-(3,4--dichlorophenyl)-5-methyl-2-pyrazoline as colorless prisms, m.p. 103-104°C.

B) A mixture of 2.5 g. of the above compound and 15.0 ml. of trifluoroacetic anhydride is allowed to stand at room temperature for one hour. The reaction mixture is filtered to collect a solid. The solid is dissolved in dichloromethane. This solution is passed through a short column of a hydrous magnesium silicate. The effluent is heated on a steam bath and hexane is added to a crystallize a product. The product is collected, then the entire crystallization procedure is repeated to give 2.50 g. of the product of the Example as needles, m.p. 150-152°C.

Example 123

N-[1-(3,4-Dichlorophenyl)-4-methyl-2-pyrazolin-3-yl]-formamide

A 2.8 g. amount of sodium metal is dissolved in 200 ml. of absolute ethanol, then 21.35 g. of 3,4-dichlorophenylhydrazine hydrochloride is added followed in 30 minutes by 6.7 g. ofmethacrylonitrile. The reaction mixture is refluxed for 5 hours, then is evaporated to dryness in vacuo. Water is added to separate a solid. The solid is collected and dissolved in dichloromethane. This solution is passed through a short column of a hydrous magneisum

silicate and recrystallized as described in Example 110 to give 16.65 g. of 3-amino-1-(3,4-dichlorophenyl)-4--methyl-2-pyrazoline as colorless prisms, m.p. 131-132.5°C.

A mixture of 3.0 g. of the preceding compound and 15 ml. of a mixture of formic acid and acetic anhydride (Feiser and Feiser, Reagents for Organic Synthesis, Vol. 1, page 4) is allowed to stand at room temperature for 1/2 hour. The reaction mixture is poured onto ice then is filtered to collect a solid. The solid is dissolved in dichloromethane and treated as described in Example 110 to give 2.72 g.of the desired product as yellow prisms, m.p. 150-152°C.

Example 124

N-[1-(3,4-Dichlorophenyl)-2-pyrazolin-3-yl]formamide

A mixture of 5.0 g. of 3-amino-1-(3,4-dichlorophenyl)-2-pyrazoline (prepared in Example 109) and 25 ml. of a mixture of formic acid and acetic anhydride (Example 123) is allowed to stand at room temperature for one hour. The resulting solid is collected by filtration and washed with hexane. The solid is dissolved in dichloromethane and is columnized and recrystallized as described in Example 113 to give 3.85 g. of the desired product as yellow prisms, m.p. 151-153°C.

Example 125

N-[1-(4-Biphenylyl)-5-methyl-2-pyrazolin-3-yl]--2,2,2-trifluoroacetamide

A 5.0 g. amount of 4-amino-biphenyl is suspended in a stirred solution of 40.0 ml. of concentrated hydrochloric acid and 27.0 ml. of water maintained at 5°C. Stirring is continued and the temperature is maintained below 10°C. during the slow addition of a solution of 8.0 g. of sodium nitrite in 16.0 ml. of water. The reaction mixture is stirred for 15 minutes longer at 5°C. then is added slowly to a cooled solution (0-5°C.) of 53.0 g. of stannous chloride in 53.0 ml. of concentrated hydrochloric acid. The resulting mixture is diluted with water and treated with a 40% solution of sodium hydroxide until alka-

line. The solid formed is collected by filtration, dissolved in dichloromethane, dried over anhydrous magnesium sulfate and filtered. The filtrate is passed through a short column of a hydrous magnesium silicate. The effluent is evaporated in vacuo to give a solid. The solid is dissolved in dichloromethane. This solution is heated to boiling and hexane is added until turbidity occurs. The mixture is cooled and filtered to give 8.5 g. of 4-biphenylhydrazine as orange crystals, m.p. 135-138°C. (dec.).

A 4.3 g. amount of the preceding compound is added to a solution of 0.1 g. of sodium metal dissolved in 100 ml. of absolute ethanol. Then 1.54 g. of crotononitrile is added and the mixture is refluxed for 6 hours and poured into water to separate a dark solid. The solid is dissolved in dichloromethane, heated to relux and recrystallized twice with hexane as previously described to give 3.6 g. of 3-amino-1-(4-biphenylyl)-5-methyl-2--pyrazoline as a solid, m.p. 174-176°C.

A 0.5 g. amount of the preceding compound is dissolved in 2.5 ml. of trifluoroacetic anhydride and allowed to remain at room temperature for 2 hours. The solvent is removed in vacuo and water is added to separate a solid. The solid is collected, dissolved in dichloromethane and is columnized and recrystallized as in Example 110 to give the product of the Example as yellow crystals, m.p. 192-193°C.

Example 126

N-(1-Phenyl-2-pyrazolin-3-yl)acetamide

A 10.0 g. amount of 3-amino-1-phenyl-2-pyrazoline (prepared in Example 116) and 10.0 ml. of acetic anhydride are mixed together and allowed to stand at room temperature for one hour. The resulting solid is collected, dissolved in dichloromethane and passed through a short column of a hydrous magnesium silicate. The effluent is heated and hexane is added to crystallize 1.2 g. of the desired product as yellow prisms, m.p. 190-191°C.

Example 127

N-[1-(*m*-Chlorophenyl)-2-pyrazolin-3-yl]-formamide

A mixture of 2.0 g. of 3-amino-1-(*m*-chlorophenyl)-2-pyrazoline (prepared in Example 118) and 10 ml. of a mixture of formic acid and acetic anhydride (Example 123) is allowed to stand at room temperature for 30 minutes. Then the solvent is evaporated *in vacuo* to give 1.32 g. of the desired product as pale yellow needles, m.p. 143-145°C.

Example 128

N-[1-(*p*-Chlorophenyl)-5-methyl-2-pyrazolin-3-yl]-formamide

A 1.72 g. amount of sodium metal is dissolved in 10 ml. of absolute ethanol, then 11.0 g. of *p*-chlorophenylhydrazine hydrochloride is added followed in 15 minutes by 4.20 g. of crotononitrile. The reaction mixture is refluxed for 18 hours. The solvent is removed *in vacuo* and water is added to the residue to separate a gum. The gum is dissolved in dichloromethane and separated from the aqueous layer. The organic layer is dried over anhydrous magnesium sulfate and filtered through a short column of a hydrous magnesium silicate. The effluent is evaporated to give a glass. A small amount of benzene and then hexane is added to the glass which gradually solidifies. The solid is collected, washed with hexane and dried to give 8.1 g. of 3-amino-1-(*p*-chlorophenyl)-5-methyl-2-pyrazoline as pink solid, m.p. 90-92°C.

A 4.8 g. amount of the above compound is dissolved in 25.0 ml. of a mixture of formic acid and acetic anhydride (Example 123). The reaction mixture is allowed to remain at room temperature for 3 hours, then ice water is added to separate a gum. The aqueous phase is decanted and the gum is dissolved in dichloromethane. The solution is dried over magnesium sulfate and passed through a hydrous magnesium silicate. The effluent is evaporated leaving a gum. The gum is dissolved in dichloromethane. The solution is heated and hexane is added until turbidity results. The mixture is cooled and filtered to yield 3.6 g. of the desired product as off-white crystals, m.p. 112-113°C.

Example 129

N-[1-(p-Chloropohenyl)-5-methyl-2-pyrazolin-3-yl)--2,2,2-trifluoroacetamide

An 8.0 g. amount of 3-amino-1-(p-chlorophenyl)-5-methyl-2-pyrazoline (prepared as described in Example 128) is dissolved in 25.0 ml. of dichloromethane with stirring. This solution is slowly added with stirring to 25.0 ml. of trifluoroacetic anhydride cooled at 10°C. The reaction mixture is stirred for 3 hours at room temperature, then the solvent is removed *in vacuo*. The residue is dissolved in dichloromethane and this solution is passed through a short column of a hydrous magnesium silicate. The effluent is concentrated while adding hexane until turbidity resluts. The solution is cooled, then filtered to give 8.5 g. of N-[1-(p-chlorophenyl)-5-methyl-2-pyrazolin-3-yl]-2,2,2-trifluoroacetamide as a white solid, m.p. 170-172°C.

Example 130

N-(1-Phenyl-2-pyrazolin-3-yl)-formamide

A mixture of 10.0 g. of 3-amino-1-phenyl-2-pyrazoline (prepared in Example 116) and 50.0 ml. of a mixture of formic acid and acetic anhydride (Example 123) is allowed to stand at room temperature for 3 hours. Water is added and the white crystals are collected by filtration.

The solid is dissolved in dichloromethane. The solution is dried over magnesium sulfate and filtered through a hydrous magnesium silicate. The filtrate is evaporated in vacuo to give pink crystals. A 500 mg. portion of crude product is recrystallized from dichloromethane-hexane to give 403 mg. of the desired product as a white solid, m.p. 140-141°C.

Example 131

N-[1-(p-Chlorophenyl)-4-methyl-2-pyrazolin-3-yl]-formamide

A 1.2 g. amount of sodium metal is dissolved in 110 ml. of absolute ethanol, then 7.5 g. of p-chlorophenylhydrazine hydrochloride is added followed by 2.9 g. of methacrylonitrile. The reaction mixture is refluxed for 18 hours. The solvent is removed in vacuo and water is added to the residue to separate a gum. The gum is collected and dissolved in dichloromethane. The organic solution is washed with water, dried over anhydrous magnesium sulfate and filtered through a short column of a hydrous magnesium silicate. The effluent is evaporated leaving a gum which solidified upon adding hexane. The solid is collected and recrystallized from ether-hexane to give 4.6 g. of 3-amino-1-(p-chlorophenyl)-4-methyl-2-pyrazoline as white crystals, m.p. 107-108°C.

A 2.2 g. amount of the preceding compound is dissolved in 10.0 ml. of a mixture of formic acid and acetic anhydride (Example 123). The mixture is allowed to remain at room temperature for 3 hours. Water is added and a yellow solid is collected by filtration. The solid is dissolved in dichloromethane. The solution is dried over anhydrous magnesium sulfate and filtered through a hydrous magnesium silicate. The filtrate is concentrated while adding hexane until turbidity occurs. The mixture is cooled and filtered to give 2.1 g. of the product of the Example as a white solid, m.p. 172°-174°C.

BAD ORIGINAL

Example 132

N-[1-(m-Chlorophenyl)-4-methyl-2-pyrazolin-3-yl]-formamide

A 3.12 g. amount of sodium metal is dissolved in 200 ml. of absolute ethanol, then 20.9 g. of m-chlorophenylhydrazine hydrochloride is added, followed by 7.6 g. of methacrylonitrile. The reaction mixture is refluxed for 18 hours. The solvent is removed in vacuo and water is added to separate a solid. The solid is collected, dissolved in dichloromethane, dried over anhydrous magnesium sulfate and filtered through a hydrous magnesium silicate. The filtrate is evaporated to give a solid. The solid is recrystallized twice from ether-hexane to give 16.2 g. of 3-amino-1-(m-chlorophenyl)-4-methyl-2--pyrazoline as white crystals, m.p. 84-85°C.

A 3.0 g. amount of the preceding product is dissolved in 10 ml. of a mixture of formic acid and acetic anhydride (Example 123). The mixture is allowed to remain at room temperature for 3 hours, then is poured into ice water to separate a partial gum and solid. This material is collected, dissolved in dichloromethane and filtered through a short column of a hydrous magnesium silicate. The effluent is concentrated while adding hexane to separate 3.1 g. of the product of the Example as light yellow crystals, m.p. 140-142°C.

Example 133

N-(5-Methyl-1-phenyl-2-pyrazolin-3-yl)-acetamide

A mixture of 2.0 g. of 3-amino-5-methyl-1-phenyl-2-pyrazoline (prepared in Example 113) 5.0 ml. of acetic anhydride and 100 mg. of 4-dimethylaminopyridine is allowed to stand at room temperature for 3 hours. The mixture is filtered to give a white solid. The solid is dissolved in dichloromethane and is columnized and recrystallized as for Example 122 (B) to give 0.90 g. of the desired product as colorless plates, m.p. 144.5-146.5°C.

Example 134

N-[1-(p-Fluorophenyl)-5-methyl-2-pyrazolin-3-yl]-acetamide

A) Sodium metal (2.8 g.) is dissolved in 200 ml. of absolute ethanol, then 16.73 g. of p-fluorophenylhydrazine hydrochloride is added followed in 10 minutes by 6.7 g. of crotononitrile. The reaction mixture is refluxed for 16 hours. The solvent is removed in vacuo and water is added to the residue to separate a solid. The solid is dissolved in dichloromethane. The solution is passed through a short column of a hydrous magnesium silicate. The effluent is refluxed and hexane is added to crystallize 11.0 g. of 3-amino-1-(p-fluorophenyl)-5-methyl-2-pyrazoline as prisms, m.p. 133-135°C.

B) A mixture of 3.0 g. of the preceding compound and 7.5 ml. of acetic anhydride is allowed to remain at room temperature for 16 hours. The mixture is filtered to collect a solid. The solid is dissolved in dichloromethane. The solution is colunmized and crystallized as for Example 122 (B) to give 1.3 g. of the product of the Example as colorless needles, m.p. 136.5-137.5°C.

Example 135

N-[5-(p-Chlorophenyl)-1-(m-fluorophenyl)-2-pyrazolin-3-yl]acetamide

A 1.4 g. amount of sodium metal is dissolved in 150 ml. of absolute ethanol, then 8.1 g. of m-flurophenylhydrazine hydrochloride is added followed in 5 minutes by 8.18 g. of p-chlorocinnamonitrile. The reaction mixture is heated at reflux for 6 hours, then is filtered and cooled. The solvent is partially evaporated and water is added to separate a semi-solid which is collected, dissolved in dichloromethane and is columnized and crystallized twice as for Example 117 to give 2.4 g. of 3-amino-5-(p-chlorophenyl)-1-(m-fluorophenyl)-2-pyrazoline as colorless needles, m.p. 135.5-136°C.

BAD ORIGINAL

A mixture of 2.5 g. of the above compound and 7.5 ml. of acetic anhydride is allowed to stand at room temperature for 16 hours. The resulting clear dark solution is poured into a mixture of 50 ml. of water, 50 ml. of ethanol and 20 ml. of 5N-sodium hydroxide. The reaction mixture is refluxed for 30 minutes on a steam bath, then is cooled and filtered to collect a solid. The solid is dissolved in dichloromethane and is columnized and crystallized as for Example 118 (B) to give 1.82 g. of the desired product as yellow needles, m.p. 182-183.5°C.

Example 136

N-[5-(p-Chlorophenyl)-1-(p-fluorophenyl)-2--pyrazolin-3-yl]acetamide

A 2.8 g. amount of sodium metal is dissolved in 100 ml. of absolute ethanol, then 16.26 g. of p-fluorophenylhydrazine hydrochloride is added followed in 5 minutes by 16.35 g. of p-chlorocinnamonitrile. The reaction mixture is refluxed for 20 hours, then the solvent is evaporated in vacuo. Water is added to separate a solid. The solid is dissolved in dichloromethane and is columnized and crystallized as for Example 113 (A) to yield 8.95 g. of 3-amino-5-(p-chlorooophenyl]-1-(p-fluorophenyl)--2-pyrazoline as a pale coral colored solid, m.p. 182-183°C.

A mixture of 4.0 g. of the preceding product and 15.0 ml. of acetic anhydride is allowed to stand at room temperature for 16 hours. Then the mixture is poured into a mixture of 50 ml. of water, 50 ml. of 95% ethanol and 55 ml. of 5N aqueous sodium hydroxide. The reaction mixture is refluxed on a steam bath for 30 minutes, then is cooled and filtered to collect a solid. The solid is dissolved in dichloromethane and is columnized and crystallized as for Example 113 (B) to yield 2.3 g. of the product of the Example as off-white needles, m.p. 133-135°C.

Example 137

N-[1-(p-Chlorophenyl)-2-pyrazolin-3-yl]-acetamide

Sodium metal (2.8 g.) is dissolved in 200 ml. of absolute ethanol, then 17.9 of p-chlorophenylhydrazine hydrochloride is added followed in 25 minutes by 5.5 g. of acrylonitrile. The reaction mixture is refluxed by 5.5 g. of acrylonitrile. The reaction mixture is refluxed for 6 hours, then is filtered hot. The filtrate is evaporated to dryness in vacuo, then water is added to separate a solid. The solid is collected by filtration, then is dissolved in dichloromethane and columnized and crystallized as for Example 118 (B) to give 8.75 g. of 3-amino-1-(p-chlorophenyl)-2-pyrazoline as colorless needles, m.p. 142.5-145°C.

A mixture of 0.5 g. of the preceding product and 1.25 ml. of acetic anhydride is allowed to remain at room temperature for 16 hours. The mixture is chilled and filtered to give 222 mg. of pale yellow crystals. The material is recrystallized from dichloromethane-hexane to give the product of the Example as a white solid, m.p. 168-170°C.

Example 138

N-[1-(p-Chlorophenyl)-4-methyl-2-pyrazolin-3-yl]-2,2,2-trifluoroacetamide

A 7.0 g. amount of 3-amino-1-(p-chlorophenyl)-4-methyl-2-pyrazoline (prepared as described in Example 131) is dissolved in 25.0 ml. of dichloromethane and cooled to 5°C. The solution is stirred and 14.0 ml. of trifluoroacetic anhydride is added. Stirring is continued at room temperature for 3 hours, then the solvent is removed in vacuo. The residue is dissolved in dichloromethane and the solution is concentrated with the addition of hexane until turbidity appears. The mixture is cooled and the solid is collected to give 5.3 g. of product. A 1.0 g. amount of this material is recrystallized from dichloromethane-hexane to give 750 mg. of the desired

product as a white solid, m.p. 181-183°C.

Example 139

N-[1-(p-Chlorophenyl)-4-methyl-2-pyrazolin-3-yl]-acetamide

A 9.0 g. amount of 3-amino-1-(p-chlorophenyl)--4-methyl-2-pyrazoline (prepared as described in Example 131) is dissolved in 54.0 ml. of acetic anhydride. The warm solution is allowed to stand for 16 hours at room temperature with some solid separation. The solid is collected by filtration and is dissolved in methanol. Then 1N potassium hydroxide in methanol is added to make basic. After 15 minutes at room temperature, the solvent is removed *in vacuo* and water is added to separate a solid. The solid is collected and recrystallized from dichloromethane-hexane to give 6.5 g. of the desired product as white crystals, m.p. 172-173°C.

Example 140

N-[1-(p-Chlorophenyl)-5-methyl-2-pyrazolin-3-yl]-acetamide

A mixture of 9.0 g. of 3-amino-1-(p-chlorophenyl)-5-methyl-2-pyrazoline (prepared as described in Example 128), 400 mg. of 4-dimethylaminopyridine and 20.0 ml. of acetic anhydride is allowed to stand at room temperature for 48 hours. The solid formed is collected by filtration, washed with cold acetic anhydride, then with hexane and is dried to give a white solid. The solid is recrystallized from dichloromethane-hexane to give 6.6 g. of the product of the Example as a white solid, m.p. 167-169°C.

Example 141

N-(4-Methyl-1-phenyl-2-pyrazolin-3-yl)-acetamide

A 10.0 g. amount of 3-amino-4-methyl-1-phenyl--2-pyrazoline (prepared as described in Example 114) is dissolved in 50 ml. of acetic anhydride, then 500 mg. of 4-dimethylaminopyridine is added and the reaction mixture is allowed to stand at room temperature for 18 hours. The mixture is poured into water to separate a gum which solidifies and is collected by filtration. The solid is added with stirring to 150 ml. of methanol containing 10.0 g. of sodium hydroxide. After 30 minutes, the solvent is removed *in vacuo* and water is added to provide a gum which becomes solid. This solid is dissolved in dichloromethane, dried over anhydrous magnesium sulfate and passed through a short column of a hydrous magnesium silicate. The effluent is concentrated while adding hexane to yield crystals. The material is collected and dried to give 3.3 g. of the desired product as a white solid, m.p. 149-150°C.

Example 142

N-[1-(p-Trifluoroacetylphenyl)-2-pyrazolin-3-yl]-acetamide

A mixture of 800 mg. of N-(1-phenyl-2-pyrazolin--3-yl)acetamide (Example 126) and 5.0 ml. of trifluoroacetic anhydride is allowed to stand at room temperature for 30 minutes. Then the mixture is evaporated to dryness *in vacuo*. The residue is dissolved in dichloromethane and is columnized and crystallized as for Example 118 (B) to yield 85 mg. of the desired product as yellow needles, m.p. 243-244°C.

Example 142

N-[1-(p-Trifluoroacetylphenyl)-2-pyrazolin-3-yl]-acetamide

A mixture of 800 mg. of N-(1-phenyl-2-pyrazolin--3-yl)acetamide (Example 126) and 5.0 ml. of trifluoroacetic anhydride is allowed to stand at room temperature for 30 minutes. Then the mixture is evaporated to dryness *in vacuo*. The residue is dissolved in dichloromethane and is columnized and crystallized as for Example 118 (B) to yield 85 mg. of the desired product as yellow needles, m.p. 243-244°C.

Example 143

N-(1-Phenyl-2-pyrazolin-3-yl)propionamide

A mixture of 10.0 g. of 3-amino-1-phenyl-2--pyrazoline (prepared as described in Example 116), 50 ml. of propionic anhydride and 200 mg. of 4-dimethylaminopyridine is kept at room temperature for 30 minutes. The mixture is cooled and filtered to give yellow crystals. The solid is dissolved in dichloromethane and is columnized and crystallized as for Example 118 (B) to yield 4.55 g. of the product of the Example as colorless crystals, m.p. 170.5-171°C.

Example 144

N-[1-(m-Fluorophenyl)-4-methyl-2-pyrazolin-3-yl]-acetamide

A 2.8 g. amount of sodium metal is dissolved in 200 ml. of absolute ethanol, then 16.2 g. of m-fluorophenylhydrazine hydrochloride is added, followed by 13.4 g. of methacrylonitrile. The reaction mixture is refluxed for 18 hours, then the solvent is evaporated *in vacuo*. Water is added to give a gum. The gum is dissolved in dichloromethane and the solution is passed through a short column of a hydrous magnesium silicate. The effluent is concentrated while adding hexane until turbidity occurs. The solution is cooled and seeded to separate a pink solid which is collected by filtration and dried to give 4.2 g. of 3-amino-1-(m-fluorophenyl)-4-methyl-2-pyrazoline.

A 4.2 g. amount of the preceding compound is dissolved in 10.0 ml. of acetic anhydride then 100 mg. of 4-dimethylaminopyridine is added and the reaction mixture is allowed to stand at room temperature for 16 hours. The mixture is poured into water to separate a gum which solidifies. The solid is collected by filtration and washed with water. The solid is dissolved in 50.0 ml. of methanol and 10.0 ml. of 1N sodium hydroxide in methanol is added. The mixture is allowed to stand at room temperature for 30 minutes, then the solvent is partly removed *in vacuo*. The addition of water separates a yellow solid. The solid is collected and dried to give 4.1 g. of the product of the Example, m.p. 171-172°C.

Example 145

N-(1-*m*-Tolyl-2-pyrazolin-3-yl)acetamide

A 2.8 g. amount of sodium metal is dissolved in 100 ml. of absolute ethanol, then 15.8 g. of *m*-tolylhydrazine hydrochloride is added followed in 5 minutes by 9.9 g. of β-ethoxypropionitrile. The reaction mixture is refluxed for 16 hours, then is evaporated to dryness *in vacuo*. Water is added to the residue to separate a solid. The solid is dissolved in dichloromethane and is columnized and crystallized as described in Example 134 (A) to give 10.1 g. of 3-amino-1-*m*-tolyl-2-pyrazoline as colorless crystals, m.p. 119-120°C.

A mixture of 3.0 g. of the above compound, 15.0 ml. of acetic anhydride and 150 mg. of 4-dimethylaminopyridine is allowed to stand at room temperature for 16 hours. The mixture is filtered to collect a small amount of solid. The solid is washed with hexane. The filtrate is allowed to stand for 16 hours and is filtered to collect additional solid. This material is washed with hexane. The solids are combined and again washed with hexane to give 1.72 g. of the desired g. of the desired product as off-white crystals, m.p. 222-225°C.

Example 146

2,2,2-Trifluoro-N-(1-phenyl-2-pyrazolin-3-yl)-acetamide

A mixture of 5.0 g. of 3-amino-1-phenyl-2-pyrazoline (prepared as described in Example 116), 200 ml. of dichloromethane, 5.0 ml. of triethylamine and 7.0 ml. of trifluoroacetic anhydride is allowed to remain at room temperature for 30 minutes. The mixture is evaporated to dryness *in vacuo*, then water is added to separate a semi-solid. The solid is dissolved in dichloromethane. The solution is dried over anhydrous sodium sulfate, columnized and recrystallized as for Example 122 (B) to give 3.0 g. of the desired product as yellow-orange prisms, m.p. 163-165°C.

Example 147

2,2,2-Trifluoro-N-[4-methyl-1-(α,α,α-trifluoro-*m*-tolyl)-2-pyrazolin-3-yl]acetamide

A 0.7 g. amount of sodium metal is dissolved in 35.0 ml. of absolute ethanol, then 5.3 g. of *m*-trifluoromethylphenylhydrazine hydrochloride is added followed slowly by the addition of 1.7 g. of methacrylonitrile. The reaction mixture is refluxed for 18 hours. The solvent is removed *in vacuo* and water is added to separate a solid. The solid is collected and dissolved in dichloromethane. The solution is dried over anhydrous magnesium sulfate then is passed through a short column of a hydrous magnesium silicate. The effluent is evaporated to give a solid. The solid is recrystallized from ether-hexane to give 3.6 g. of (±)-3-amino-4-methyl-1-(α,α,α-trifluoro-*m*-tolyl)-2-pyrazoline as white crystals.

A 7.9 g. amount of the preceding compound (prepared as described above) is dissolved in 25.0 ml. of dichloromethane, then 15.8 ml. of trifluoroacetic anhydride is added and the mixture is stirred at room temperature for 2 1/2 hours. The excess anhydride is removed *in vacuo* and the reaction is repeated using hexane as the medium. The reaction mixture is again evaporated *in vacuo* and the

resulting tan solid is dissolved in ether and is filtered through a 200 mesh synthetic magnesium silicate adsorbent. The filtrate is concentrated while adding hexane to precipitate white crystals. The mixture is cooled for 16 hours and filtered to yield 8.4 g. of the desired product, m.p. 121-122°C.

Example 148

N-(1,5-Diphenyl-2-pyrazolin-3-yl)formamide

A mixture of 5.0 g. of 3-amino-1,5-diphenyl-2--pyrazoline (prepared as described in Example 112) and 25.0 ml. of a mixture of formic acid and acetic anhydride (Example 123) is allowed to remain at room temperature for 30 minutes. Then hexane is added and the mixture is filtered. The solid obtained is dissolved in dichloromethane. The solution is columnized and recrystallized as described in Example 122 (B) to yield 3.55 g. of the product of the Example as off-white needles, m.p. 158-160°C.

Example 149

2,2,2-Trifluoro-N-[1-(p-fluorophenyl)-2-pyrazolin--3-yl]acetamide

Sodium metal (2.8 g.) is dissolved in 100 ml. of absolute ethanol, then 16.26 g. of p-fluorophenylhydrazine hydrochloride is added followed in 5 minutes by 5.5 g. of acrylonitrile. The reaction mixture is refluxed for 7 hours, then the solvent is evaporated near dryness and water is added to separate a solid. The solid is dissolved in dichloromethane. The solution is columnized and the product is recrystallized as for Example 113 (A) to give 6.6 g. of 3-amino-1-(p-fluorophenyl)-2-pyrazoline as colorless needles, m.p. 114-115°C.

A mixture of 4.15 g. of the preceding product; 100 ml. of dichloromethane and 10.0 ml. of trifluoroacetic anhydride is allowed to remain at room temperature for 30 minutes. The mixture is evaporated to dryness *in vacuo*. The resulting solid iws washed with water, then is dissolved in dichloromethane. The solution is columnized and the desired product is recrystallized as for Example 122 (A)

to yield 5.45 g. as pale yellow prisms, m.p. 145.5-147°C.

Example 150

2,2,2-Trifluoro-N-[1-(4-trifluoroacetyl-*m*-tolyl)--2-pyrazolin-3-yl]acetamide

A mixture of 4.0 g. of 3-amino-1-*m*-tolyl-2-pyrazoline (prepared in Example 145) and 20.0 ml. of trifluoroacetic anhydride is stirred at room temperature giving an exothermic reaction resulting in a green solution. The solution is cooled to provide a precipitate. The solvent is evaporated *in vacuo* to give a solid. The solid is dissolved in dichloromethane. The solution is columnized and the desired product is recrystallized as described in Example 134 (A) to give 3.9 g. of the product of the Example as yellow needles, 171.5-173°C.

Example 151

2,2,2-Trifluoro-N-[1-(*p*-fluorophenyl)-5-phenyl-2--pyrazolin-3-yl]acetamide

Sodium metal (2.8 g.) is dissolved in 100 ml. of absolute ethanol, then 16.2 g. of *p*-fluorophenylhydrazine hydrochloride is added followed in 5 minutes by 12.9 g. of cinnamonitrile. The reaction mixture is refluxed for 5 hours. Then the solvent is evaporated *in vacuo*. Water is added to separate a solid. The solid is dissolved in dichloromethane and is columnized and recrystallized 3 times as described in Example 134 (A) to give 6.2 g. of 3-amino--1-(*p*-fluorophenyl)-5-phenyl-2-pyrazoline as buff colored needles, m.p. 160-161°C.

A mixture of 4.0 g. of the preceding compound, 50 ml. of dichloromethane and 10 ml. of trifluoroacetic anhydride is allowed to remain at room temperature for 30 minutes. The solvent is evaporated *in vacuo*. The residual solid is washed with water and dried. The solid is dissolved in dichloromethane. The solution is columnized and the desired product is recrystallized as described in Example 134 (A) to give 4.36 g. as colorless matted needles, m.p. 143-143.5°C.

Example 152

2,2,2-Trifluoro-N-[1-(p-fluorophenyl)-5-methyl-2--pyrazolin-3-yl]acetamide

An 8.4 g. amount of sodium metal is dissolved in 420 ml. of absolute ethanol, then 48.8 g. of p-fluorophenylhydrazine hydrochloride is added, followed in 5 minutes by 20.1 g. of crotononitrile. The reaction mixture is refluxed for 16 hours. The solvent is removed *in vacuo* and water is added to separate a solid. The solid is collected by filtration and dissolved in dichloromethane. The solution is columnized and a product is recrystallized as for Example 134 (A) to give 25.5 g. of 3-amino-1-(p-fluorophenyl)-5-methyl-2-pyrazoline as prisms, m.p. 135-136°C.

A mixture of 10.0 g. of the preceding product, 50 ml. of dichloromethane and 25 ml. of trifluoroacetic anhydride is allowed to remain at room temperature for 30 minutes. The solvent is removed *in vacuo* and water is added to separate a solid. The solid is collected and washed with a saturated sodium bicarbonate solution. The solid is dissolved in dichloromethane. The solution is columnized and recrystallized twice as described in Example 134 (A) to give 11.15 g. of the product of the Example as yellow crystals, m.p. 116-117°C.

Example 153

N-[1-(p-Fluorophenyl)-5-methyl-2-pyrazolin-3-yl]-propionamide

A mixture of 3.0 g. of 3-amino-1-(p-fluorophenyl)-5-methyl-2-pyrazoline (prepared in Example 152) and 5.0 ml. of propionic anhydride is allowed to remain at room temperature for 2 hours. The reaction mixture is poured into water and the water is decanted. This step is repeated. The precipitate is treated with saturated sodium bicarbonate solution then is filtered. The solid is dissolved in dichloromethane. The solution is columnized and the desired product is recrystallized as described in Example 134 (A) to give 2.77 g. as colorless

plates, m.p. 125-126.5°C.

Example 154

N-[1-(p-Fluorophenyl)-4-methyl-2-pyrazolin-3-yl]-formamide

A 2.8 g. amount of sodium metal is dissolved in 150 ml. of absolute ethanol, then 16.26 g. of p-fluorophenylhydrazine hydrochloride is added followed in 5 minutes by 6.7 g. of methacrylonitrile. The reaction mixture is refluxed for 8 hours, then the solvent is removed in vacuo. Water is added to the residue to separate an oil. The oil crystallizes on standing for 16 hours. The solid is dissolved in dichloromethane and is columnized and recrystallized twice as described in Example 134 (A) to give 8.65 g. of 3-amino-1-(p-fluorophenyl)-4-methyl--2-pyrazoline as colorless prisms.

A mixture of 3.3 g. of the above compound and 15.0 ml. of a mixture of formic acid and acetic anhydride (Example 123) is allowed to remain at room temperature for 16 hours. Water is added and the mixture is filtered. The solid collected is recrystallized from acetone-hexane to give 2.65 g. of the desired product as pale yellow prisms, m.p. 166-168.5°C.

Example 155

N-(5-Methyl-1-phenyl-2-pyrazolin-3-yl)propionamide

A mixture of 5.0 g. of 3-amino-5-methyl-1-phenyl-2-pyrazoline (prepared in Example 113) and 25.0 ml. of propionic anhydride is allowed to remain at room temperature for 16 hours. The reaction mixture is poured into water to separate an oil which crystallizes. The solid is collected by filtration and dissolved in dichloromethane. The solution is passed through a short column of a hydrous magnesium silicate. The effluent is heated at reflux and hexane is added. The first heavy precipitate (purple color) is discarded. The solution is cooled and the ensuing precipitate is collected by filtration. The solid is again dissolved in dichloromethane, columnized and recrystallized with hexane to give 2.3 g. of the product of the Example as plates, m.p. 96.0-96.5°C.

Example 156

N-[1-(p-Fluorophenyl)-5-phenyl-2-pyrazolin-3-yl)-formamide

A mixture of 5.0 g. of 3-amino-1-(p-fluorophenyl)-5-phenyl-2-pyrazoline (prepared as described in Example 151) and 85.0 ml. of a mixture of formic acid and acetic anhydride (Example 123) is allowed to remain at room temperature for 2 hours. The solvent is evaporated *in vacuo* to give an oil. Water is added to separate a solid. The solid is dissolved in dichloromethane and is columnized and recrystallized twice as described in Example 134 (A) to give 3.55 g. of the desired product as colorless crystals, m.p. 158.5-160.5°C.

Example 157

N-[5-(p-Chlorophenyl)-1-(m-fluorophenyl)-2-pyrazolin-3-yl]formamide

A) Sodium metal (1.4 g.) is dissolved in 150 ml. of absolute ethanol. The solution is cooled in a cold water bath, then 8.1 g. of m-fluorophenylhydrazine hydrochloride is added followed in 5 minutes by 8.18 g. of p-chlorocinnamonitrile. The reaction mixture is heated at reflux for 6 hours then is filtered. The filtrate is cooled and water is added. The semi-solid is collected by filtration, dissolved in dichloromethane and is columnized and recrystallized twice as described in Example 134 (A) to give 2.4 g. of 3-amino-5-(p-chlorophenyl)-1-(m-fluorophenyl)-2-pyrazoline as colorless needles, m.p. 135.5-136°C.

B) A mixture of 2.0 g. of the preceding compound and 10.0 ml. of a mixture of formic acid and acetic anhydride (Example 123) is allowed to remain at room temperature for 2 hours. The mixture is poured into water to separate an oil which becomes semi-solid. The material is decanted, then water is added and the solid is collected by filtration. The solid is dissolved in dichloromethane and is columnized and recrystallized as described

in Example 134 (A) to yield 1.02 g. of the desired product as off-white needles, m.p. 128.5-130.5°C.

Example 158

N-[5-(p-Chlorophenyl)-1-phenyl-2-pyrazolin-3-yl]-formamide

A mixture of 500 ml. of absolute ethanol, 5.0 ml. of 50% choline in methanol, 32.4 g. of phenylhydrazine and 49.08 g. of p-chlorocinnamonitrile is refluxed for 7 hours, then is allowed to stand at room temperature for 16 hours. The solvent is evaporated in vacuo. The solid is dissolved in dichloromethane and the solution is columnized and recrystallized twice as described in Example 134 (A) to give 12.0 g. of 3-amino-5-(p-chlorophenyl)-1-phenyl-2-pyrazoline as colorless needles, m.p. 183.5-185.5°C.

A mixture of 10.0 g. of the preceding product and 100 ml. of a mixture of formic acid and acetic anhydride (Example 123) is allowed to remain at room temperature for 2.5 hours. The mixture is poured into water to separate a semi-solid. The procedure of Example 157 (B) is followed to yield 6.3 g. of the desired product as yellow prisms, m.p. 135-136°C.; resolidifies, then melts, m.p. 149-150°C.

Example 159

N-[1-(p-Chlorophenyl)-5-phenyl-2-pyrazolin-3-yl]-formamide

A 5.52 g. amount of sodium metal is dissolved in 300 ml. of absolute methanol. The solution is cooled in a cold water bath, then 35.8 g. of p-chlorophenylhydrazine hydrochloride is added followed in 5 minutes by 25.9 g. of cinnamonitrile. After 2 hours, the mixture is cooled to give a precipitate. Water is added and the mixture is extracted with dichloromethane. The organic layer is dried, then is columnized and recrystallized as described in Example 134 (A) to give 22.5 g. of crude product. A 5.0 g. amount of this material is recrystallized as above to give 3.35 g. of 3-amino-1-(p-chloro-

phenyl)-5-phenyl-2-pyrazoline as colorless needles, m.p. 159-161°C.

A mixture of 15.0 g. of the above crude product and 85.0 ml. of a mixture of formic acid and acetic anhydride (Example 123) is allowed to remain at room temperature for 2 hours. The reaction mixture is poured into water and filtered to collect the solid. The solid is washed with a solution of saturated sodium bicarbonate, then water. The material is dried, dissolved in dichloromethane, then is columnized and recrystallized as above to give 12.0 g. of the desired product as pale yellow crystals. A 5.0 g. amount of this material is recrystallized as above to give 4.70 g. of the desired product, m.p. 164-166°C.

Example 160

N-(1,5-Diphenyl-2-pyrazolin-3-yl)propionamide

A mixture of 10.0 g. of 3-amino-1,5-diphenyl-2--pyrazoline (prepared in Example 112), 30.0 ml. of propionic anhydride and 500 mg. of 4-dimethylaminopyridine is heated on a steam bath for 3 hours. The mixture is poured into water to separate an oil. The oil crystallizes and the solid is collected by filtration. The solid is dissolved in dichloromethane. The solution is dried over anhydrous sodium sulfate then is columnized and recrystallized twice as for Example 134 (A) to give 6.0 g. of the product of the Example as colorless plates, m.p. 148.0-148.5°C.

Example 161

N-[1-(p-Chlorophenyl)-5-phenyl-2-pyrazolin-3-yl]--2,2,2-trifluoroacetamide

A mixture of 2.75 g. of 3-amino-1-(p-chlorophenyl)-5-phenyl-2-pyrazoline (prepared in Example 159) and 10.0 ml. of trifluoroacetic anhydride is allowed to remain at room temperature for 16 hours. The solvent is evaporated *in vacuo* and the residue is dissolved in dichloromethane. The solution is columnized and recrystallized as for Example 134 (A) to give 2.35 g. of the desired product as pale yellow crystals, m.p. 176-178°C.

Example 162

N-(1-Phenyl-5-p-tolyl-2-pyrazolin-3-yl)-propionamide

A mixture of 4.5 g. of 3-amino-1-phenyl-5-p-tolyl-2-pyrazoline (prepared in Example 111), 20.0 ml. of propionic anhydride and 200 mg. of 4-dimethylaminopyridine is heated on a steam bath for 2 hours. The reaction mixture is cooled to room temperature then water is added to separate a solid. The solid is collected then is dissolved in dichloromethane. The solution is passed through a short column of a hydrous magnesium silicate. The effluent is heated at reflux and hexane is added until turbidity appears. The mixture is cooled and filtered to collect a solid. The solid is recrystallized again from 80% ethanol. This material is dissolved in 100 ml. of methanol, then 200 mg. of potassium carbonate is added and the mixture is refluxed for 15 minutes. Water is added until turbidity appears. The mixture is cooled and filtered to collect the solid. This solid is dissolved dichloromethane. This solution is columnized and recrystallized as described above to yield 2.4 g. of the desired product as colorless needles, m.p. 123.5-125°C.

Example 163

N-[1,5-Bis(p-chlorophenyl)-2-pyrazolin-3-yl]formamide

A 0.86 g. amount of sodium metal is dissolved in 100 ml. of absolute ethanol, then 7.0 g. of p-chlorophenylhydrazine hydrochloride is added followed in 30 minutes by 5.5 g. of p-chlorocinnamonitrile. The reaction mixture is refluxed for 16 hours then the solvent is removed *in vacuo*. Water is added to separate a solid. The solid is collected and dissolved in dichloromethane. The solution is passed through a short column of a hydrous magnesium silicate. The effluent is heated and hexane is added. The first oily material coming out of solution is removed by filtration. The filtrate is cooled to give a precipitate which is collected and dissolved in acetone.

The solution is treated with activated charcoal and filtered. Hexane is added to the filtrate to crystallize a product. This material is recrystallized from acetone--hexane to give 1.48 g. of 3-amino-1,5-bis(p-chlorophenyl)--2-pyrazoline as colorless needles, m.p. 149-150°C.

A mixture of 1.85 g. of 3-amino-1,5-bis(p-chlorophenyl)-2-pyrazoline (prepared as described above) and 10.0 ml. of a mixture of formic acid and acetic anhydride (Example 123) is allowed to remain at room temperature for 2 hours, then water is added and the mixture is filtered to collect a semi-solid. The solid is dissolved in dichloromethane and the solution is columnized and recrystallized twice as for Example 134 (A) to yield 1.10 g. of the product of the Example as off-white crystals, m.p. 198-200°C.

## Example 164

## N-[1-(2-Benzothiazolyl)-5-p-tolyl-2-pyrazollin-3-yl]-propionamide

A) A 500 mg. amount of sodium metal is dissolved in 250 ml. of absolute ethanol, then 8.3 g. of 2-hydrazinobenzothiazole is added followed by 7.4 g. of 4-methylcinnamonitrile. The reaction mixture is refluxed for 4 hours then is cooled. The precipitate formed is collected by filtration and washed with ethanol, then water, to give 13.7 g. of crude product. This material is recrystallized from 2-methoxyethanol, filtered and washed with hexane then methanol to yield 8.55 g. of 2-(3-amino-5-p-tolyl-2--pyrazolin-1-yl)benzothiazole as colorless prisms, m.p. 282-285°C.

B) A mixture of 3.5 g. of the preceding product, 150 mg. of 4-dimethylaminopyridine and 25 ml. of propionic anhydride is refluxed for 2 hours. The reaction mixture is then poured into water and this mixture is allowed to stand at room temperature for 16 hours. The separated solid is collected by filtration. The solid is dissolved in dichloromethane. The organic solution is passed through a short column of a hydrous magnesium silicate. The effluent is concentrated while adding hexane until turbidity results. The solution is cooled, then filtered to collect a solid. The solid is again dissolved in dichloromethane, columnized and recrystallized as described above to give 2.45 g. of the desired product as colorless prisms, m.p. 208-209°C.

Example 165

N-[1-(2-Benzothiazolyl)-5-phenyl-2-pyrazolin-3-yl]-acetamide

A 460 mg. amount of sodium metal is dissolved in 150 ml. of absolute ethanol, then 16.5 g. of 2-hydrazinobenzothiazole is added, followed by 12.9 g. of cinnamonitrile. The reaction mixture is refluxed for 16 hours, then is cooled. Some of the solvent is removed *in vacuo* and the mixture is filtered. The precipitate is washed with hexane to give 25.67 g. of 2-(3-amino-5-phenyl-2--pyrazolin-1-yl)benzothiazole as tan crystals.

A mixture of 3.95 g. of the preceding product, 200 mg. of 4-dimethylaminopyridine and 25 ml. of acetic anhydride is refluxed for 2 hours. Then the solution is cooled and the solvent is evaporated *in vacuo* to give a solid. The solid is recrystallized from acetone/hexane. The recrystallized product is dissolved in dichloromethane. This solution is columnized and recrystallized as described in Example 164 (B) to yield 1.95 g. of the product of the Example as pale yellow prisms, m.p. 220-222°C.

Examples 166-177

Following substantially the procedure set out in Example 73 the various hydrazino compounds is condensed with acrylonitrile to produce the corresponding Example title compound.

| Example | Example Title Compound | Hydrazine |
|---|---|---|
| Example 166 | 2-(3-amino-2-pyrazolin-1-yl)-3,5-dichloropyridine | 3,5-dichloro-2-hydrazino-pyridine |
| Example 167 | 2-(3-amino-2-pyrazolin-1-yl)-3-methylquinoxaline | 2-hydrazino-3-methylquinox-aline |
| Example 168 | 2-(3-amino-2-pyrazolin-1-yl)-4,6-dimethyl-*s*-triazine | 2-hydrazino-4,6-dimethyl-*s*-triazine |
| Example 169 | 2-(3-amino-2-pyrazolin-1-yl)-benzoxoazole | 2-hydrazino-benzoxazole |
| Example 170 | 2-(3-amino-2-pyrazolin-1-yl)-5-chloro-3-nitropyridine | 5-chloro-2-hydrazino-3-nitropyridine |
| Example 171 | 1-(3-amino-2-pyrazolin-1-yl)-isoquinoline | 1-hydrazinoiso-quinoline |
| Example 172 | 6-(3-amino-2-pyrazolin-1-yl)-purine | 6-hydrazino-purine |
| Example 173 | 2-(3-amino-2-pyrazolin-1-yl)-4,6-dimethylpyrimidine | 2-hydrazino-4,6-dimethyl-pyrimidine |
| Example 174 | 2-(3-amino-2-pyrazolin-1-yl)-3-chloropyridine | 3-chloro-2-hydrazinopyri-dine |
| Example 175 | 2-(3-amino-2-pyrazolin-1-yl)-6-bromopyridine | 2-bromo-6-hydrazinopyri-dine |
| Example 176 | 2-(3-amino-2-pyrazolin-1-yl)-6-methoxypyridine | 2-hydrazino-6-methoxypyri-dine |
| Example 177 | 2-(3-amino-2-pyrazolin-1-yl)-2-thiazoline | 2-hydrazino-thiazole |

Example 178

2-[3-Amino-5-(p-chlorophenyl)-2-pyrazolin-1-yl]-
-6-chloropyridine

An 0.3 g, amount of sodium metal is dissolved in 100 ml. of absolute ethanol, then 7.2 g. of 2-chloro-6-hydrazinopyridine (Example 73) is added, followed by 8.2 g. of 4-chlorocinnamonitrile. The reaction mixture is refluxed for 18 hours, then is evaporated *in vacuo* to a gum. Water is added to give a solid. The solid is collected and dissolved in dichloromethane, dried over magnesium sulfate and filtered. The filtrate is concentrated while adding hexane to separate yellow crystals on cooling. The product is collected and washed with ether-hexane to give 10.3 g. of tan solid. The solid is dissolved in dichloromethane, filtered through absorbent magnesium silicate, then is concentrated while adding hexane to give the product of the Example as white crystals, m.p. 157-158°C.

Example 179

2-(3-Amino-5-phenyl-2-pyrazolin-1-yl)-6-
-chloropyridine

As for Example 73, a mixture of 0.23 g. of sodium metal, 50 ml. of absolute ethanol, 7.11 g. of 2-chloro-6-hydrazinopyridine and 6.5 g. of cinnamonitrile yields 7.35 g. of crude product. The crude material is dissolved in dichloromethane, filtered through hydrous magnesium silicate and concentrated while adding hexane to give 5.90 g. of the desired product as off-white crystals, 187.5-188.5°C.

WE CLAIM:

1. A compound of the formula:

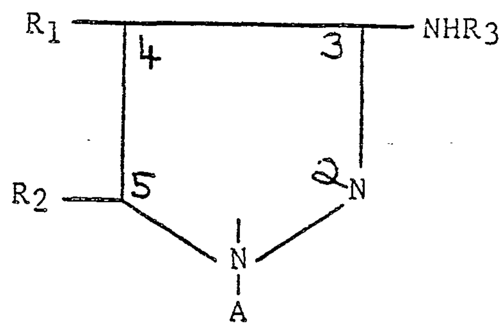

I

or a pharmacologically acceptable acid-addition salt thereof, wherein when A is

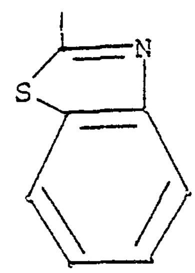

;

then $R_1$ is H; $R_2$ is phenyl or p-tolyl; and $R_3$ is $-CO-R_4$ where $R_4$ is $C_1$-$C_4$ alkyl; or when A is

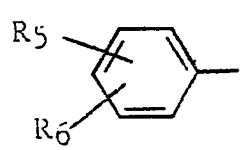

where $R_5$ and $R_6$ are the same or different and are H, Cl, F, $C_1$-$C_4$ alkyl, $-CF_3$ or $COCF_3$; then $R_1$ is H or $C_1$-$C_4$

alkyl; $R_2$ is H, $C_1$-$C_4$ alkyl, phenyl or

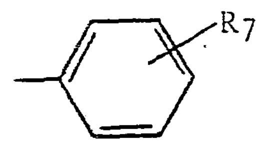

where $R_7$ is halogen; and $R_3$ is -CHO, $-COCF_3$ or $-COR_8$ where $R_8$ is $C_1$-$C_4$ alkyl; or when A is

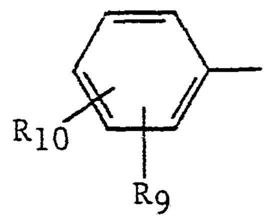

where $R_9$ and $R_{10}$ are the same or different and are H or halogen with the proviso that they cannot both be H; then $R_1$ is H or $C_1$-$C_4$ alkyl; $R_2$ is H, $C_1$-$C_4$ alkyl, phenyl or

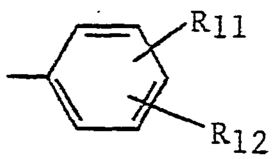

where $R_{11}$ and $R_{12}$ are the same or different and are H, halogen or $C_1$-$C_4$ alkyl with the proviso that they cannot both be H; and $R_3$ is H; or when A is

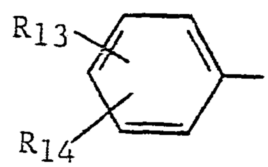

where $R_{13}$ and $R_{14}$ are H, Cl or F; then $R_1$ and $R_2$ are H or $C_1$-$C_4$ alkyl; and $R_3$ is $C_1$-$C_4$ alkyl, $CH_2CF_3$ or

$$-CH=\underset{}{\overset{\displaystyle COOC_2H_5\\|}{C}}-COOC_2H_5$$; or when A is 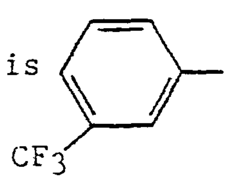 ;

then $R_1$ is H or $C_1$-$C_4$ alkyl; $R_2$ is $C_1$-$C_4$ alkyl or phenyl; and $R_3$ is H; or when A is

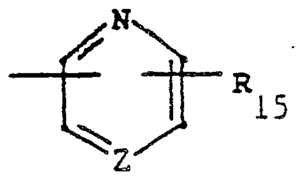 or 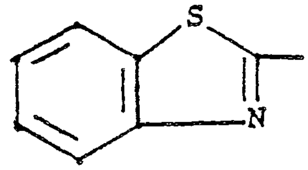

where Z is N or CH and $R_{15}$ is H, halogen or $C_1$-$C_4$ alkyl; then $R_1$ and $R_2$ are the same or different and are H, $C_1$-$C_4$ alkyl, phenyl or substituted phenyl; and $R_3$ is H with the proviso that when $R_1$ and $R_2$ are both H; then A can also be:

(1) an alkoxy-, dihalo- or nitro, halo-substituted pyridine; or

(2) a heterocycle consisting of

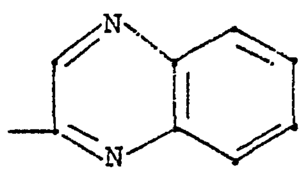 ; 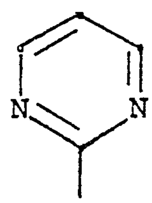 ; 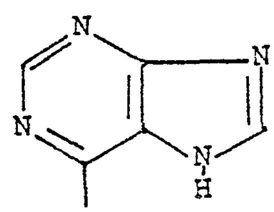 ;

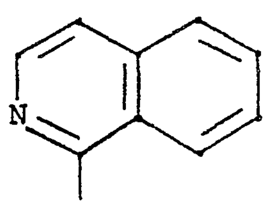 ; 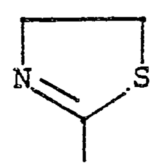 ; 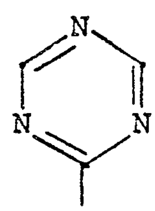 ; or

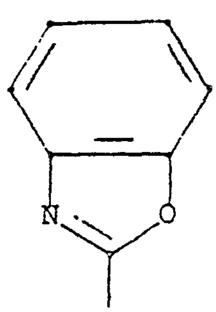

and the methyl substituted above heterocycles; or when A is

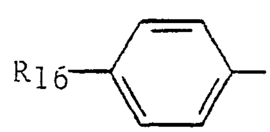

where $R_{16}$ is H, $C_1$-$C_4$ alkyl, $HO_2C$-, $CH_3O$-, $-COCF_3$ or phenyl; then $R_1$ is H or $C_1$-$C_4$ alkyl; $R_2$ is H, $C_1$-$C_4$ alkyl, phenyl or 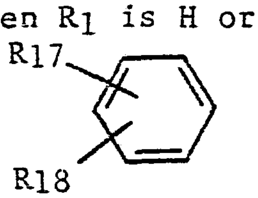

where $R_{17}$ and $R_{18}$ are the same or different and are H, halogen or $C_1$-$C_4$ alkyl with the proviso that they cannot both be H; and $R_3$ is H; or when A is

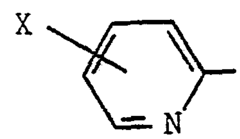

where X is halogen or $-CF_3$; then $R_1$ and $R_2$ are H or $C_1$-$C_4$ alkyl; and $R_3$ is $-COCH_3$ or -CHO.

2. A composition of matter in dosage unit form useful for meliorating the inflammation and/or the progressive joint deterioration characteristic of arthritic disease in mammals, preventing the onset of asthmatic symptoms and allergic diseases in mammals, or as an analgesic, antibacterial or antifungal agent in mammals which comprises from about 0.5 milligram to about 100 milligrams per kilogram of body weight per daily dose of a compound of the formula as defined in Claim 1 and a pharmaceutically acceptable carrier.

3. A composition of matter in dosage unit form which comprises an effective analgesic amount of a compound of the formula as a quaternary salt:

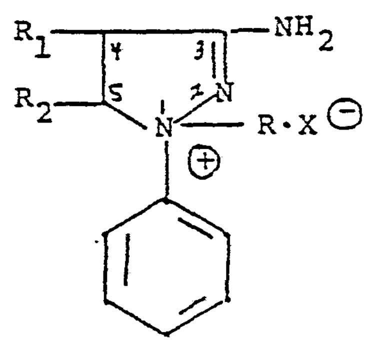

wherein R is $C_1$-$C_4$ alkyl; $R_1$ and $R_2$ are hydrogen or $C_1$-$C_2$ alkyl; and X is halogen; and a pharmaceutically acceptable carrier.

European Patent Office

# EUROPEAN SEARCH REPORT

0070376

Application number

EP 82 10 4682

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | US-A-2 726 248 (J.D. KENDALL et al.) * Whole document * | 1 | C 07 D 231/06<br>C 07 D 401/04<br>C 07 D 417/04<br>C 07 D 403/04<br>C 07 D 473/04<br>A 61 K 31/415<br>A 61 K 31/44<br>A 61 K 31/495<br>A 61 K 31/53 //<br>C 07 D 213/77<br>C 07 D 241/20 |
| X | GB-A-1 324 687 (CHINOIN) * Claims * | 1 | |
| X | EP-A-0 022 578 (WELLCOME) * Claims * | 1-3 | |
| E | EP-A-0 055 418 (WELLCOME) * Claims * | 1-3 | |
| X | CHEMICAL ABSTRACTS, vol. 54, no. 2, 25th January 1960, column 1501b-f, Columbus, Ohio, USA A.N. KOST et al.: "Reactions of hydrazine derivatives. XXII. 3-Amino-1-arylpyrazolines and their salicylidene derivatives" & ZHUR. OBSHCHEI KHIM. 29, 498-502 (1959) * Abstract * | 1 | **TECHNICAL FIELDS SEARCHED (Int. Cl. 3)**<br>C 07 D 231/00<br>C 07 D 401/00<br>C 07 D 417/00<br>C 07 D 403/00 |

The present search report has been drawn up for all claims

| Place of search | Date of completion of the search | Examiner |
|---|---|---|
| THE HAGUE | 10-11-1982 | CREMERS K. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82